# EUROPEAN PATENT APPLICATION

(11) **EP 4 357 780 A1**
(43) Date of publication of application: **24.04.2024**
(21) Application number: 22825053.6
(22) Date of filing: 16.06.2022
(51) Int. Cl.: G01N 33/569, C07K 14/08, C07K 16/10, C12M 1/34, C12N 15/40, C12Q 1/37, C12Q 1/70, G01N 33/53

(54) **SARS-COV-2 IMMUNOASSAY METHOD AND IMMUNOASSAY KIT, AND MONOCLONAL ANTIBODY OR ANTIBODY FRAGMENT THEREOF**

(30) Priority: 16.06.2021 JP 2021100117
(71) Applicant: Sekisui Medical Co., Ltd., Tokyo 103-0027 (JP)
(72) Inventor: HIROTA, Jiro, Tokyo 103-0027 (JP); UNO, Satoru, Tokyo 103-0027 (JP); OCHIAI, Yasushi, Tokyo 103-0027 (JP); YAJI, Shohei, Tokyo 103-0027 (JP); HEWSON, Christopher Kenta, Tokyo 103-0027 (JP); KOHNO, Keigo, Tokyo 103-0027 (JP); ASAI, Tomohide, Tokyo 103-0027 (JP)
(74) Representative: Ter Meer Steinmeister & Partner
(86) International application number: PCT/JP2022/024133
(87) International publication number: WO 2022/265065

(57) **Abstract**

The present invention provides an immunoassay method for assaying SARS-CoV-2, including a step of contacting a biological sample with a monoclonal antibody or an antibody fragment thereof that binds to a nucleocapsid protein of SARS-CoV-2, wherein the monoclonal antibody or the antibody fragment thereof binds to the SARS-CoV-2 treated with a serine protease.

## Description

### [Technical Field]

The present invention relates to an immunoassay method and an immunoassay kit for assaying SARS-CoV-2. The present invention also relates to a monoclonal antibody or an antibody fragment thereof.

Priority is claimed on Japanese Patent Application No. 2021-100117, filed June 16, 2021, the contents of which are incorporated herein by reference.

### [Background Art]

SARS-CoV-2 is a virus that causes the coronavirus disease 2019 (COVID-19). SARS-CoV-2 belongs to the family Coronaviridae, which is a family of viruses each having a single-stranded, positive-strand RNA as its genome. Currently, the corona virus disease 2019 is prevalent all over the world, and many cases of infection have been confirmed.

PCR tests and antigen tests are used to detect SARS-CoV-2. The results of the antigen test are available in about 10 minutes after sample collection, so that the antigen test can be performed more easily than the PCR test. On the other hand, a highly sensitive and specific test is required for rapid detection of SARS-CoV-2. However, the antigen test is generally less sensitive than the PCR test. Therefore, there is a need for more sensitive antigen tests.

Analysis of SARS-CoV-2 samples taken from human bodies has also been performed (Non-Patent Literature 1). However, it was still unclear what sequence or what sample of SARS-CoV-2 is a suitable target analyte that enables highly sensitive antigen testing.

### [Citation List]

### [Non Patent Literature]

Non-Patent Literature 1: Journal of proteome research 2020, 19, 4389-4392 Mass Spectrometric Identification of SARS-CoV-2 Proteins from Gargle Solution Samples of COVID-19 Patients
Non-Patent Literature 2: Package insert for in-vitro diagnostic reagent "Espline (registered trademark) SARS-CoV-2" (Fujirebio Inc.)
Non-Patent Literature 3: Package insert for in-vitro diagnostic reagent "QuickNavi (registered trademark)-COVID19 Ag" (Denka Company Limited)

### [Summary of Invention]

### [Technical Problem]

An object of the present invention is to provide a SARS-CoV-2 immunoassay kit and a SARS-CoV-2 immunoassay method that enable highly sensitive and rapid immunoassay. Another object of the present invention is to provide a monoclonal antibody or an antibody fragment thereof that can be used in the immunoassay kit and immunoassay method.

### [Solution to Problem]

The present inventors have made intensive studies to solve the above problems. As a result, the present inventors have also found that the above problems can be solved by using a monoclonal antibody or an antibody fragment thereof that binds to SARS-CoV-2 treated with a serine protease, and have completed the present invention.

In the Examples, the present inventors compare the sensitivity of one embodiment of the immunoassay method of the present invention with the sensitivities of Espline (registered trademark) SARS-CoV-2 (Fujirebio Inc.) (Non-Patent Literature 2) and QuickNavi (registered trademark)-COVID19 Ag (Denka Company Limited) (Non-Patent Literature 3), which are commercially available reagents for SARS-CoV-2 antigen detection. The embodiment of the immunoassay method of the present invention exhibits sensitivity comparable or superior to these two reagents with a shorter measurement time.

Specifically, the present invention is as follows.

[1] An immunoassay method for assaying SARS-CoV-2, including contacting a biological sample with a monoclonal antibody or an antibody fragment thereof that binds to a nucleocapsid protein of SARS-CoV-2,
   wherein the monoclonal antibody or the antibody fragment thereof binds to SARS-CoV-2 treated with a serine protease.
[2] The immunoassay method according to [1], wherein the monoclonal antibody or the antibody fragment thereof reacts with an amino acid sequence of 3 88th to 414th amino acids of SEQ ID NO: 21.
[3] The immunoassay method according to [1] or [2], wherein the monoclonal antibody or the antibody fragment thereof recognizes an epitope in an amino acid sequence represented by KQQTVTLPAADLDDFSK (SEQ ID NO: 1) or an epitope in an amino acid sequence represented by AADLDDFSKQLQ (SEQ ID NO: 2).
[4] The immunoassay method according to any one of [1] to [3], wherein the biological sample is treated with a serine protease.
[5] The immunoassay method according to any one of [1] to [4], wherein the biological sample is a respiratory secretion.
[6] The immunoassay method according to any one of [1] to [5], wherein the monoclonal antibody or the antibody fragment includes two types of monoclonal antibodies or antibody fragments thereof that bind to peptide fragments of a nucleocapsid protein of SARS-CoV-2, wherein:
   the two types of monoclonal antibodies or antibody fragments thereof are a first monoclonal antibody or an antibody fragment thereof and a second monoclonal antibody or an antibody fragment thereof, wherein the first monoclonal antibody or the antibody fragment thereof has a labeling substance indirectly or directly bound thereto, and the second monoclonal antibody or the antibody fragment thereof is indirectly or directly bound to a solid phase; and
   the first monoclonal antibody or the antibody fragment thereof and the second monoclonal antibody or the antibody fragment thereof recognize different epitopes.
[7] The immunoassay method according to [6], wherein, as step (1), the biological sample is contacted with the first monoclonal antibody or the antibody fragment thereof having the labeling substance bound thereto, thereby forming a first complex, and which further includes step (2) of contacting the first complex with the second monoclonal antibody or the antibody fragment thereof to form a second complex, and step (3) of measuring a signal derived from the labeling substance.
[8] The immunoassay method according to [6] or [7], wherein the first monoclonal antibody or the antibody fragment thereof is a monoclonal antibody or an antibody fragment thereof that recognizes an epitope in the amino acid sequence represented by KQQTVTLLPAADLDFSK (SEQ ID NO: 1), and the second monoclonal antibody or the antibody fragment thereof is a monoclonal antibody or an antibody fragment thereof that recognizes an epitope in the amino acid sequence represented by AADLDDFSKQLQ (SEQ ID NO: 2).
[9] The immunoassay method according to any one of [1] to [8], which is immunochromatography or ELISA.
[10] The immunoassay method according to any one of [1] to [9], wherein the monoclonal antibody or the antibody fragment thereof is at least one selected from the group consisting of:
   (1) an antibody or an antibody fragment thereof that comprises a heavy chain variable region comprising CDR1 having an amino acid sequence of SEQ ID NO: 3, CDR2 having an amino acid sequence of SEQ ID NO: 4, and CDR3 having an amino acid sequence of SEQ ID NO: 5, and a light chain variable region comprising CDR1 having an amino acid sequence of SEQ ID NO: 6, CDR2 having an amino acid sequence of SEQ ID NO: 7, and CDR3 having an amino acid sequence of SEQ ID NO: 8;
   (2) an antibody or an antibody fragment thereof that comprises a heavy chain variable region comprising CDR1 having an amino acid sequence of SEQ ID NO: 9, CDR2 having an amino acid sequence of SEQ ID NO: 10, and CDR3 having an amino acid sequence of SEQ ID NO: 11, and a light chain variable region comprising CDR1 having an amino acid sequence of SEQ ID NO: 12, CDR2 having an amino acid sequence of SEQ ID NO: 13, and CDR3 having an amino acid sequence of SEQ ID NO: 14;
   (3) an antibody or an antibody fragment thereof that comprises a heavy chain variable region comprising CDR1 having an amino acid sequence of SEQ ID NO: 15, CDR2 having an amino acid sequence of SEQ ID NO: 16, and CDR3 having an amino acid sequence of SEQ ID NO: 17, and a light chain variable region comprising CDR1 having an amino acid sequence of SEQ ID NO: 18, CDR2 having an amino acid sequence of SEQ ID NO: 19, and CDR3 having an amino acid sequence of SEQ ID NO: 20; and
   (4) an antibody or an antibody fragment thereof that comprises a heavy chain variable region and a light chain variable region respectively having 80 % or more amino acid sequence identity with the heavy chain variable region and the light chain variable region of the antibody or the antibody fragment thereof in any one of (1) to (3).
[11] The immunoassay method according to any one of [1] to [10], wherein the measurement sensitivity in an assay performed with respect to a SARS-CoV-2 antigen treated with 1 µg/mL of trypsin for 30 minutes at 37 °C is 60 % or more, preferably 70 % or more, more preferably 80 % or more, even more preferably 90 % or more, as compared to the measurement sensitivity in an assay performed with respect to a SARS-CoV-2 antigen before the trypsin treatment.
[12] An immunoassay kit for assaying SARS-CoV-2 in a biological sample, including a monoclonal antibody or an antibody fragment thereof that binds to a nucleocapsid protein of SARS-CoV-2, wherein the monoclonal antibody or the antibody fragment thereof binds to the SARS-CoV-2 treated with a serine protease.
[13] The immunoassay kit according to [12], wherein the monoclonal antibody or the antibody fragment thereof reacts with an amino acid sequence of 388th to 414th amino acids of SEQ ID NO: 21.
[14] The immunoassay kit according to [12] or [13], wherein the monoclonal antibody or the antibody fragment thereof recognizes an epitope in an amino acid sequence represented by KQQTVTLPAADLDDFSK (SEQ ID NO: 1) or an epitope in an amino acid sequence represented by AADLDDFSKQLQ (SEQ ID NO: 2).
[15] The immunoassay kit according to claim any one of [12] to [14], wherein the biological sample is treated with a serine protease.
[16] The immunoassay kit according to any one of [12] to [15], wherein the biological sample is a respiratory secretion.
[17] The immunoassay kit according to any one of [12] to [16], wherein the monoclonal antibody or the antibody fragment includes two types of monoclonal antibodies or antibody fragments thereof that bind to peptide fragments of a nucleocapsid protein of SARS-CoV-2, wherein:
   the two types of monoclonal antibodies or antibody fragments thereof are a first monoclonal antibody or an antibody fragment thereof and a second monoclonal antibody or an antibody fragment thereof, wherein the first monoclonal antibody or the antibody fragment thereof has a labeling substance indirectly or directly bound thereto, and the second monoclonal antibody or the antibody fragment thereof is indirectly or directly bound to a solid phase; and
   the first monoclonal antibody or the antibody fragment thereof and the second monoclonal antibody or the antibody fragment thereof recognize different epitopes.
[18] The immunoassay kit according to [17], wherein the first monoclonal antibody or the antibody fragment thereof is a monoclonal antibody or an antibody fragment thereof that recognizes an epitope in the amino acid sequence represented by KQQTVTLLPAADLDFSK (SEQ ID NO: 1), and the second monoclonal antibody or the antibody fragment thereof is a monoclonal antibody or an antibody fragment thereof that recognizes an epitope in the amino acid sequence represented by AADLDDFSKQLQ (SEQ ID NO: 2).
[19] The immunoassay kit according to any one of [12] to [18], which is a kit for immunochromatography or ELISA.
[20] The immunoassay kit according to any one of [12] to [19], wherein the monoclonal antibody or the antibody fragment thereof is at least one selected from the group consisting of:
   (1) an antibody or an antibody fragment thereof that comprises a heavy chain variable region comprising CDR1 having an amino acid sequence of SEQ ID NO: 3, CDR2 having an amino acid sequence of SEQ ID NO: 4, and CDR3 having an amino acid sequence of SEQ ID NO: 5, and a light chain variable region comprising CDR1 having an amino acid sequence of SEQ ID NO: 6, CDR2 having an amino acid sequence of SEQ ID NO: 7, and CDR3 having an amino acid sequence of SEQ ID NO: 8;
   (2) an antibody or an antibody fragment thereof that comprises a heavy chain variable region comprising CDR1 having an amino acid sequence of SEQ ID NO: 9, CDR2 having an amino acid sequence of SEQ ID NO: 10, and CDR3 having an amino acid sequence of SEQ ID NO: 11, and a light chain variable region comprising CDR1 having an amino acid sequence of SEQ ID NO: 12, CDR2 having an amino acid sequence of SEQ ID NO: 13, and CDR3 having an amino acid sequence of SEQ ID NO: 14;
   (3) an antibody or an antibody fragment thereof that comprises a heavy chain variable region comprising CDR1 having an amino acid sequence of SEQ ID NO: 15, CDR2 having an amino acid sequence of SEQ ID NO: 16, and CDR3 having an amino acid sequence of SEQ ID NO: 17, and a light chain variable region comprising CDR1 having an amino acid sequence of SEQ ID NO: 18, CDR2 having an amino acid sequence of SEQ ID NO: 19, and CDR3 having an amino acid sequence of SEQ ID NO: 20; and
   (4) an antibody or an antibody fragment thereof that comprises a heavy chain variable region and a light chain variable region respectively having 80 % or more amino acid sequence identity with the heavy chain variable region and the light chain variable region of the antibody or the antibody fragment thereof in any one of (1) to (3).
[21] The immunoassay kit according to any one of [12] to [20], wherein the measurement sensitivity in an assay performed with respect to a SARS-CoV-2 antigen treated with 1 µg/mL of trypsin for 30 minutes at 37 °C is 60 % or more, preferably 70 % or more, more preferably 80 % or more, even more preferably 90 % or more, as compared to the measurement sensitivity in an assay performed with respect to a SARS-CoV-2 antigen before the trypsin treatment.
[22] A monoclonal antibody or an antibody fragment thereof that binds to a nucleocapsid protein of SARS-CoV-2, wherein the monoclonal antibody or the antibody fragment thereof binds to SARS-CoV-2 treated with a serine protease.
[23] The monoclonal antibody or the antibody fragment thereof according to [22], which recognizes an epitope in an amino acid sequence represented by KQQTVTLLPAADLDDFSK (SEQ ID NO: 1) or an epitope in an amino acid sequence represented by AADLDDFSKQLQ (SEQ ID NO: 2).
[24] The monoclonal antibody or the antibody fragment thereof according to [22] or [23], which is selected from the group consisting of:
   (1) an antibody or an antibody fragment thereof that comprises a heavy chain variable region comprising CDR1 having an amino acid sequence of SEQ ID NO: 3, CDR2 having an amino acid sequence of SEQ ID NO: 4, and CDR3 having an amino acid sequence of SEQ ID NO: 5, and a light chain variable region comprising CDR1 having an amino acid sequence of SEQ ID NO: 6, CDR2 having an amino acid sequence of SEQ ID NO: 7, and CDR3 having an amino acid sequence of SEQ ID NO: 8;
   (2) an antibody or an antibody fragment thereof that comprises a heavy chain variable region comprising CDR1 having an amino acid sequence of SEQ ID NO: 9, CDR2 having an amino acid sequence of SEQ ID NO: 10, and CDR3 having an amino acid sequence of SEQ ID NO: 11, and a light chain variable region comprising CDR1 having an amino acid sequence of SEQ ID NO: 12, CDR2 having an amino acid sequence of SEQ ID NO: 13, and CDR3 having an amino acid sequence of SEQ ID NO: 14;
   (3) an antibody or an antibody fragment thereof that comprises a heavy chain variable region comprising CDR1 having an amino acid sequence of SEQ ID NO: 15, CDR2 having an amino acid sequence of SEQ ID NO: 16, and CDR3 having an amino acid sequence of SEQ ID NO: 17, and a light chain variable region comprising CDR1 having an amino acid sequence of SEQ ID NO: 18, CDR2 having an amino acid sequence of SEQ ID NO: 19, and CDR3 having an amino acid sequence of SEQ ID NO: 20; and
   (4) an antibody or an antibody fragment thereof that comprises a heavy chain variable region and a light chain variable region respectively having 80 % or more amino acid sequence identity with the heavy chain variable region and the light chain variable region of the antibody or the antibody fragment thereof in any one of (1) to (3).
[25] A complex including a first antibody or an antibody fragment thereof, a second antibody or an antibody fragment thereof, and a SARS-CoV-2 nucleocapsid protein, wherein the first antibody or the antibody fragment thereof, and the second antibody or the antibody fragment thereof are each the antibody or the antibody fragment thereof according to any one of [22] to [24].

The present invention also includes the following embodiments.
(A) The immunoassay method according to any one of [5] to [11], wherein the first monoclonal antibody or the antibody fragment thereof is a monoclonal antibody or an antibody fragment thereof that recognizes an epitope in an amino acid sequence represented by LLPAA (SEQ ID NO: 25), and the second monoclonal antibody or the antibody fragment thereof is a monoclonal antibody or an antibody fragment thereof that recognizes an epitope in an amino acid sequence represented by LDDFSKQLQ (SEQ ID NO: 26).
(B) The immunoassay kit according to any one of [17] to [21], wherein the first monoclonal antibody or the antibody fragment thereof is a monoclonal antibody or an antibody fragment thereof that recognizes an epitope in an amino acid sequence represented by LLPAA (SEQ ID NO: 25), and the second monoclonal antibody or the antibody fragment thereof is a monoclonal antibody or an antibody fragment thereof that recognizes an epitope in an amino acid sequence represented by LDDFSKQLQ (SEQ ID NO: 26).
(C) The monoclonal antibody or the antibody fragment thereof according to [19], which recognizes an amino acid sequence represented by LLPAA (SEQ ID NO: 25) as an epitope, or recognizes an epitope in an amino acid sequence represented by LDDFSKQLQ (SEQ ID NO: 26).

### [Advantageous Effects of Invention]

The present invention can provide a SARS-CoV-2 immunoassay kit and a SARS-CoV-2 immunoassay method that enable highly sensitive and rapid immunoassay, as well as a monoclonal antibody or an antibody fragment thereof that can be used therein.

### [Brief Description of Drawings]

FIG. 1 is diagram showing the amino acid sequence of a nucleocapsid protein.
FIG. 2 is diagram showing the corresponding relationship between the amino acid sequence of a synthetic peptide used for epitope analysis for an antibody and the amino acid sequence of a nucleocapsid protein.
FIG. 3 is a diagram showing the relationship between the time of trypsin treatment of SARS-CoV-2 antigen and the measurement sensitivity.
FIG. 4 is a diagram showing the relationship between the time of nasal swab treatment of SARS-CoV-2 nucleocapsid protein and the measurement sensitivity.

### [Description of Embodiments]

### Method of immunoassay for SARS-CoV-2

### (Biological sample)

Examples of the "biological sample" in the present specification include solid tissues and body fluids derived from living bodies.

More specific examples of biological samples include blood, serum, plasma, urine, tears, ear discharge, prostatic fluid, and respiratory secretions. Respiratory secretions are preferred. In the context of the present specification, the term "respiratory secretion" means bodily fluids secreted in or on the tissues of the respiratory organs. Examples of respiratory secretions include bodily fluids secreted in the nostrils, nasal cavities, pharynx, nasopharynx, oral cavity, trachea, bronchi, and lungs, and particularly preferred examples include nasopharyngeal swabs, nasal swabs, saliva, and sputum. In the context of the present specification, "respiratory organs" is a generic term for organs related to respiration, and covers organs ranging from the nasal vestibule to the alveoli (lungs) via the nasal cavity, pharynx, larynx, trachea, bronchi, and bronchioles.

Examples of subjects from which the biological sample is to be collected include humans or animals (e.g., monkeys, dogs, and cats), of which humans are preferable. The biological sample may be a biological sample as it is taken from a subject, or may be a sample obtained by subjecting a collected biological sample to treatments such as dilution and concentration that are usually performed. The person who collects and prepares a biological sample used in the present invention may or may not be identical to the person who performs the immunoassay method of the present invention. Further, the biological sample used in the immunoassay method of the present invention may be one collected or prepared during implementation of the immunoassay method of the present invention, or one previously collected or prepared and stored.

As the biological sample, it is preferable to use a biological sample collected from a site where a gene encoding a serine protease is expressed, for example, a swab of the surface of the biological sample. After the biological sample is collected from a living body, the biological sample may be treated with a serine protease. In the context of the present specification, the biological sample being treated with a serine protease means either that the biological sample is one collected from a site where a gene encoding a serine protease is expressed, or that a biological sample having been collected from a living body is treated with a serine protease.

The "serine protease" is a protease having a serine residue that executes a nucleophilic attack as a catalytic residue, and an enzyme that hydrolyzes peptide bonds on the carboxyl side of L-arginine and L-lysine.

Organs such as tracheal epithelium are known to have serine proteases expressed, such as TMPRSS2, TMPRSS4 and TMPRSS 11D. It is assumed that SARS-CoV-2-derived proteins degraded by serine proteases are also present in biological samples collected from organs such as the tracheal epithelium. In the present invention, SARS-CoV-2-derived nucleocapsid proteins degraded by serine proteases can be detected. Specifically, it is possible to detect peptide fragments produced by degradation of the nucleocapsid protein of SARS-CoV-2 by a serine protease. Therefore, the present invention can avoid reduction in measurement sensitivity, which is caused by decomposition of the SARS-CoV-2 antigen by serine proteases in vivo, thereby allowing SARS-CoV-2 to be detected with high sensitivity. The measurement sensitivity for the SARS-CoV-2 antigen in the present invention may be 60 % or more after enzymatic treatment with a serine protease such as trypsin, as compared to that before the enzymatic treatment. For example, when an assay is performed with respect to a SARS-CoV-2 antigen treated with 1 µg/mL of trypsin for 30 minutes at 37 °C, the measurement sensitivity is 60 % or more, preferably 70 % or more, more preferably 80 % or more, even more preferably 90 % or more, as compared to the measurement sensitivity in an assay performed with respect to a SARS-CoV-2 antigen before the trypsin treatment.

Epitopes contained in proteins derived from SARS-CoV-2, which correspond to specific antibodies, may be degraded by serine proteases. When the amino acid sequence of an epitope is cleaved by a serine protease, this specific corresponding antibody loses reactivity to SARS-CoV-2 derived proteins. This means that the specific antibody can no longer detect SARS-CoV-2, or the detection sensitivity for SARS-CoV-2 with the specific antibody decreases. In addition, when detecting a protein derived from SARS-CoV-2 by a sandwich system using two antibodies that recognize different epitopes, even if the amino acid sequence of the epitope is not cleaved, a portion existing between the two epitopes may be cleaved by a serine protease. In such a case, the SARS-CoV-2-derived proteins cannot be sandwiched between the two antibodies, so that the detection system using these antibodies can longer detect SARS-CoV-2, or the detection sensitivity for SARS-CoV-2 with this detection system decreases. In the present invention, SARS-CoV-2 degraded by serine proteases can be detected by using two antibodies capable of sandwiching peptide fragments generated from SARS-CoV-2 degraded by serine proteases. Therefore, the risk of false negative in SARS-CoV-2 detection due to the above causes can be reduced.

In the context of the present specification, the term "epitope" means a portion of an antigen (SARS-CoV-2 in the case of the present invention), which is recognized by an antibody.

### (SARS-CoV-2)

SARS-CoV-2 is a virus that causes the coronavirus disease 2019 (COVID-19). SARS-CoV-2 virus particles are composed of four proteins known as the spike protein, nucleocapsid protein, membrane protein, and envelope protein, as well as RNA.

The SARS-CoV-2 nucleocapsid protein is a protein composed of 419 amino acids (SEQ ID NO: 21). Mutations can occur in the nucleocapsid protein and therefore the nucleocapsid protein contains peptide fragments having amino acid sequences with at least 90 % identity (e.g., 91 %, 92 %, 93 %, 94 %, 95 %, 96 %, 97 %, 98 %, or 99 % identity) with the amino acid sequence represented by SEQ ID NO: 21.

The nucleocapsid protein contains an NTD antigen (containing the N-terminal RNA-binding domain), a CTD antigen (containing the C-terminal dimerization domain), and a Ser/Arg (SR)-rich linker domain located in the middle.

The NTD antigen is a region with amino acids 1 to 174 of the nucleocapsid protein. Among them, the region with the 1st to 49th amino acids is the N-terminal region, and the region with the 50th to 174th amino acids is the RNA binding domain.

The amino acid sequence of the NTD antigen is shown below.

MSDNGPQNQRNAPRITFGGPSDSTGSNQNGERSGARSKQRRPQGLPNNTASWF

TALTQHGKEDLKFPRGQGVPINTNSSPDDQIGYYRRATRRIRGGDGKMKDLSPR
WYFYYLGTGPEAGLPYGANKDGIIWVATEGALNTPKDHIGTRNPANNAAIVLQ
LPQGTTLPKGFYAE (SEQ ID NO: 22)

The linker domain is a region with amino acids 175 to 247 of the nucleocapsid protein.

The amino acid sequence of the linker domain is shown below.

GSRGGSQASSRSSSRSRNSSRNSTPGSSRGTSPARMAGNGGDAALALLLLDRLN

QLESKMSGKGQQQQGQTVT (SEQ ID NO: 23)

The CTD antigen is a region with amino acids 248 to 419 of the nucleocapsid protein. Among them, the region with the 248th to 364th amino acids is the dimerization domain, and the region with the 365th to 419th amino acids is the C-terminal region.

The amino acid sequence of the CTD antigen is shown below.

KKSAAEASKKPRQKRTATKAYNVTQAFGRRGPEQTQGNFGDQELIRQGTDYKH
WPQIAQFAPSASAFFGMSRIGMEVTPSGTWLTYTGAIKLDDKDPNFKDQVILLN
KHIDAYKTFPPTEPKKDKKKKADETQALPQRQKKQQTVTLLPAADLDDFSKQL
QQSMSSADSTQA (SEQ ID NO: 24)

### (Monoclonal antibody)

In the context of the present specification, the term "monoclonal antibody" refers to an antibody or antibody molecule that is obtained from clones derived from a single antibody-producing cell. In the immunoassay method of the present invention, an antibody fragment possessing the function of monoclonal antibody can also be used as long as the effects of the present invention can be achieved. Examples of antibody fragments possessing the function of monoclonal antibody include a functional fragment including the Fab portion of a monoclonal antibody obtained by enzymatic digestion of the monoclonal antibody, a functional fragment including the Fab portion of a monoclonal antibody produced by genetic recombination, a functional fragment including scFv produced by phage display method, and the like.

### (Peptide fragment of nucleocapsid protein of SARS-CoV-2)

The monoclonal antibody or the antibody fragment thereof used in the immunoassay method of the present invention binds to SARS-CoV-2 treated with a serine protease. Hereinbelow, the monoclonal antibody or the antibody fragment thereof that binds to SARS-CoV-2 treated with a serine protease is also referred to as the monoclonal antibody of the present invention.

A first embodiment of the monoclonal antibody of the present invention is capable of recognizing an epitope in the region with amino acids 365 to 419 of the CTD antigen in the nucleocapsid protein, preferably an epitope in an amino acid sequence represented by SEQ ID NO: 1, which is present in the C-terminal region, more preferably an amino acid sequence represented by LLPAA (SEQ ID NO: 25) as an epitope.

A second embodiment of the monoclonal antibody of the present invention is capable of recognizing an epitope in the region with amino acids 365 to 419 of the CTD antigen in the nucleocapsid protein, preferably an epitope in an amino acid sequence represented by SEQ ID NO: 2, which is present in the C-terminal region, more preferably an amino acid sequence represented by LDDFSKQLQ (SEQ ID NO: 26) as an epitope.

The monoclonal antibody of the invention may be an isolated monoclonal antibody or an antibody fragment thereof.

The monoclonal antibody of the present invention preferably specifically recognizes an epitope in a peptide fragment of the nucleocapsid protein of SARS-CoV-2 that is generated upon degradation by serine proteases. In this context, "specifically recognizes an epitope" means that the monoclonal antibody or antibody fragment thereof does not substantially bind to anything other than a specific peptide fragment or epitope.

The first embodiment of the monoclonal antibody of the present invention more preferably recognizes none of the following amino acid sequences as an epitope: an amino acid sequence in the NTD antigen, an amino acid sequence in the linker domain, and an amino acid sequence in a region with the 248th to 364th amino acids, which is the dimerization domain in the CTD antigen.

The second embodiment of the monoclonal antibody of the present invention more preferably recognizes none of the following amino acid sequences as an epitope: an amino acid sequence in the NTD antigen, an amino acid sequence in the linker domain, and an amino acid sequence in a region with the 248th to 364th amino acids, which is the dimerization domain in the CTD antigen.

In order to determine whether the monoclonal antibody of the present invention "does not substantially bind" to a peptide fragment having a certain amino acid sequence, for example, it is possible to perform a measurement based on the SPR method, using Biacore (registered trademark) T100 or T200, while having the monoclonal antibody of the present invention being immobilized. Whether the monoclonal antibody "does not substantially bind" can also be determined by other methods or means well known to those skilled in the art than the above SPR method.

In the C-terminal region of the nucleocapsid protein, a serine protease is thought to cleave the nucleocapsid protein between K (lysine) located at the end of the amino acid sequence represented by SEQ ID NO: 1 and Q (glutamine) located at the beginning of the amino acid sequence represented by QLQQSMSSA (SEQ ID NO: 27). Presumably, this results in formation of a peptide fragment with the amino acid sequence represented by SEQ ID NO: 1. It is assumed that the monoclonal antibody of the invention is capable of binding to this peptide fragment.

Further, considering the results of the epitope analysis for the S32217 antibody described in a later section, a sample treated with a serine protease may also contain a peptide fragment having further additional amino acids on the C-terminal side of the peptide fragment having the amino acid sequence represented by SEQ ID NO: 1 (specifically, a peptide fragment having an amino acid sequence LDDFSKQLQ (SEQ ID NO: 26)).

The immunoassay for SARS-CoV-2 can be performed with only a single type of the monoclonal antibody of the present invention by, for example, adopting the competitive method described below. However, it is preferable to use two types of the monoclonal antibodies of the present invention for performing the immunoassay for SARS-CoV-2. In this instance, the two monoclonal antibodies preferably recognize different epitopes. To recognize different epitopes in this context means that the amino acid sequences recognized as epitopes by the two monoclonal antibodies do not overlap. Using "two types" of the monoclonal antibodies of the present invention requires no more than using "at least two types" of the monoclonal antibodies of the present invention, and does not exclude embodiments using more than two types of the monoclonal antibodies of the present invention from the scope of the present invention.

When two types of the monoclonal antibodies of the present invention are used, it is preferable that the two types of the monoclonal antibodies recognize different epitopes in the amino acid sequence represented by KQQTVTLLPAADLDDFSKQLQ (SEQ ID NO: 28), it is more preferable that one of the monoclonal antibodies recognizes an epitope in the amino acid sequence represented by SEQ ID NO: 1, while the other monoclonal antibody recognizes an epitope in the amino acid sequence represented by SEQ ID NO: 2, and it is even more preferable that one of the monoclonal antibodies recognizes the amino acid sequence represented by LLPAA (SEQ ID NO: 25) as an epitope, while the other monoclonal antibody recognizes an epitope in the amino acid sequence represented by LDDFSKQLQ (SEQ ID NO: 29).

The monoclonal antibody or antibodies of the present invention is/are preferably any one or two selected from the group consisting of (1) to (4) below.

(1) An antibody or an antibody fragment thereof that comprises a heavy chain variable region comprising CDR1 having an amino acid sequence of SEQ ID NO: 3, CDR2 having an amino acid sequence of SEQ ID NO: 4, and CDR3 having an amino acid sequence of SEQ ID NO: 5, and a light chain variable region comprising CDR1 having an amino acid sequence of SEQ ID NO: 6, CDR2 having an amino acid sequence of SEQ ID NO: 7, and CDR3 having an amino acid sequence of SEQ ID NO: 8.
(2) An antibody or an antibody fragment thereof that includes a heavy chain variable region comprising CDR1 having an amino acid sequence of SEQ ID NO: 9, CDR2 having an amino acid sequence of SEQ ID NO: 10, and CDR3 having an amino acid sequence of SEQ ID NO: 11, and a light chain variable region comprising CDR1 having an amino acid sequence of SEQ ID NO: 12, CDR2 having an amino acid sequence of SEQ ID NO: 13, and CDR3 having an amino acid sequence of SEQ ID NO: 14.
(3) An antibody or an antibody fragment thereof that includes a heavy chain variable region comprising CDR1 having an amino acid sequence of SEQ ID NO: 15, CDR2 having an amino acid sequence of SEQ ID NO: 16, and CDR3 having an amino acid sequence of SEQ ID NO: 17, and a light chain variable region comprising CDR1 having an amino acid sequence of SEQ ID NO: 18, CDR2 having an amino acid sequence of SEQ ID NO: 19, and CDR3 having an amino acid sequence of SEQ ID NO: 20.
(4) An antibody or an antibody fragment thereof that includes a heavy chain variable region and a light chain variable region respectively having 80 % or more amino acid sequence identity with the heavy chain variable region and the light chain variable region of the antibody or the antibody fragment thereof in any one of (1) to (3), wherein the amino acid sequence identity is preferably 90 % or more, more preferably 95 % or more, even more preferably 98 % or more.

The amino acid sequence identity described above is preferably a sequence identity relative to the amino acid sequence that constitutes CDR1, CDR2 and CDR3 in the heavy chain variable region as well as CDR1, CDR2, and CDR3 in the light chain variable region (i.e., HCCDR1 + HCCDR2 + HCCDR3 + LCCDR1 + LCCDR2 + LCCDR3).

In the above context, "CDR1 having an amino acid sequence of SEQ ID NO: 3" means that the CDR1 consists of an amino acid sequence of SEQ ID NO: 3. The same applies to other CDR's (complementarity-determining regions).

The monoclonal antibody of the present invention may include a constant region (C region) as well as the heavy chain variable region and the light chain variable region. As the constant region, a CL region may be included in the light chain, and a CH region (CH1, CH2, and CH3) may be included in the heavy chain.

The monoclonal antibody or antibodies of the present invention is/are preferably any one or two selected from the group consisting of (1), (2), and (4) below.

(1) An antibody or an antibody fragment thereof that includes a heavy chain variable region comprising CDR1 having an amino acid sequence of SEQ ID NO: 3, CDR2 having an amino acid sequence of SEQ ID NO: 4, and CDR3 having an amino acid sequence of SEQ ID NO: 5, and a light chain variable region comprising CDR1 having an amino acid sequence of SEQ ID NO: 6, CDR2 having an amino acid sequence of SEQ ID NO: 7, and CDR3 having an amino acid sequence of SEQ ID NO: 8.
(2) An antibody or an antibody fragment thereof that includes a heavy chain variable region comprising CDR1 having an amino acid sequence of SEQ ID NO: 9, CDR2 having an amino acid sequence of SEQ ID NO: 10, and CDR3 having an amino acid sequence of SEQ ID NO: 11, and a light chain variable region comprising CDR1 having an amino acid sequence of SEQ ID NO: 12, CDR2 having an amino acid sequence of SEQ ID NO: 13, and CDR3 having an amino acid sequence of SEQ ID NO: 14.
(4) An antibody or an antibody fragment thereof that includes a heavy chain variable region and a light chain variable region respectively having 80 % or more amino acid sequence identity with the heavy chain variable region and the light chain variable region of the antibody or the antibody fragment thereof in any one of (1) and (2), wherein the amino acid sequence identity is preferably 90 % or more, more preferably 95 % or more, even more preferably 98 % or more.

When two types of the monoclonal antibodies of the present invention are used, the two types of the monoclonal antibodies are preferably the antibody (1) and the antibody (2). It is most preferable to use the antibody (1) as a labeled antibody and the antibody (2) as a solid-phase antibody.

Examples of antibodies similar to the antibody (1) include antibodies that recognize an epitope in the amino acid sequence represented by SEQ ID NO: 1, such as S32213 antibody and S32212 antibody. Further, examples of the antibody (1) include antibodies that recognize the amino acid sequence represented by LLPAA (SEQ ID NO: 25) as an epitope, such as S32213 antibody.

Examples of antibodies similar to the antibody (2) include antibodies that recognize an epitope in the amino acid sequence AADLDDFSKQLQQSMSSA (SEQ ID NO: 30), such as S32217 antibody and S32209 antibody. Further, examples of the antibody (2) include antibodies that recognize an epitope in the amino acid sequence represented by LDDFSKQLQ (SEQ ID NO: 29), such as S32217 antibody.

Examples of the antibody (3) include antibodies that recognize an epitope in the C-terminal region of the CTD antigen, such as S32223 antibody. The S32223 antibody is presumed to have similar reactivity as the S32217 antibody.

The present specification includes descriptions indicating that a monoclonal antibody or an antibody fragment thereof "reacts with" "recognizes" or "binds to" a specific substance or amino acid sequence, which however are used interchangeably. Whether or not a monoclonal antibody "reacts with" an antigen (compound) can be determined through antigen-immobilized ELISA, competitive ELISA, sandwich ELISA, or the like. Alternatively, a method using the principle of surface plasmon resonance (SPR method) can also be used. The SPR method can be performed using equipment, sensors and reagents commercially available under the name Biacore (registered trademark).

For example, when the same procedure as the epitope analysis (antibody-epitope analysis 1) in Example 1 described below is followed, it can be appreciated that the amino acid sequence contained in the peptide fragment with the highest absorbance has been recognized as an epitope.

### (Method for preparing monoclonal antibody)

The monoclonal antibody of the present invention can be prepared by dissolving a full-length nucleocapsid protein as an antigen (immunogen) in a solvent such as phosphate-buffered saline, and administering the resulting solution to a non-human animal for immunization. Immunization may be performed using an emulsion after addition of an appropriate adjuvant to the solution as necessary. Examples of adjuvants include generally used adjuvants, such as water-in-oil emulsions, water-in-oil-in-water emulsions, oil-in-water emulsions, liposomes, and aluminum hydroxide gels, as well as proteins or peptide substances derived from biological components. For example, Freund's incomplete adjuvant or Freund's complete adjuvant can be suitably used. The administration route, dosage, and administration time for the adjuvant are not particularly limited, but are preferably selected appropriately so as to enhance the desired immune response in the animal to be immunized with the antigen.

The type of animal used for immunization is also not particularly limited, and mammals such as mice, rats, cows, rabbits, goats, sheep, or alpacas are preferred, and mice or rats are more preferred. Animals can be immunized following common techniques, for example, by subcutaneously, intracutaneously, intravenously, or intraperitoneally injecting an antigen solution, preferably a mixture thereof with an adjuvant, into the animals. Since the immune response generally differs depending on the type and strain of the animal to be immunized, it is desirable to appropriately set the immunization schedule according to the animal used. It is preferable to repeat the antigen administration several times after the initial immunization.

For obtaining the monoclonal antibody of the present invention, the following operations may be subsequently performed, which, however, are not essential and do not limit the present invention. Methods for producing monoclonal antibodies per se are well known and widely used in the art, and those skilled in the art can prepare the monoclonal antibody of the present invention by using the antigens as described above (see, for example, Antibodies, A Laboratory Manual (Cold Spring Harbor Laboratory Press, (1988), Chapter 6, etc.).

After the final immunization, antibody-producing spleen cells or lymph node cells are extracted from the immunized animal and fused with a myeloma-derived cell line having high proliferative potential to prepare hybridomas. Cells with high antibody-producing ability (in terms of quality and quantity) are preferably used for cell fusion, and it is more preferable that the myeloma-derived cell line is compatible with the animal from which the antibody-producing cells to be fused are derived. Cell fusion can be performed according to methods known in the art. For example, a polyethylene glycol method, a method using Sendai virus, a method using electric current, or the like can be employed. The resulting hybridomas can be grown according to conditions commonly used in the art. A desired hybridoma can be selected while checking the properties of the antibody to be produced. Hybridoma cloning can be performed by well-known methods such as the limiting dilution method and the soft agar method.

After the cloning step, the ability of the produced monoclonal antibody to bind to the full-length nucleocapsid protein can be assayed by methods such as ELISA, RIA, and immunofluorescence. These operations allow determination of whether the selected hybridomas produce monoclonal antibodies with the desired properties.

By mass-culturing the hybridomas selected as described above, monoclonal antibodies having the desired properties can be produced. The method for mass-culturing is not particularly limited, and examples thereof include a method that cultures hybridomas in an appropriate medium to produce monoclonal antibodies in the medium, and a method that injects hybridomas into the peritoneal cavity of a mammal to allow the hybridomas to proliferate, thereby producing monoclonal antibodies in ascites.

As the monoclonal antibody of the present invention, it is possible to use an antibody fragment of a monoclonal antibody having antigen-antibody reactivity as well as the whole antibody molecule. In addition to those obtained through the process of immunizing animals as described above, it is also possible to use monoclonal antibodies obtained using gene recombination techniques, such as chimeric antibodies, humanized antibodies and human antibodies. Examples of fragments of monoclonal antibodies include F(ab')₂, Fab', scFv, and the like. These fragments can be prepared by treating the monoclonal antibody obtained as described above with a protease (e.g., pepsin or papain), or cloning the DNA of the antibody and expressing it in a culture system using E. coli or yeast.

A sandwich system can also be constructed in the immunoassay method of the present invention by using two types of monoclonal antibodies that bind to peptide fragments of the nucleocapsid protein of SARS-CoV-2. A sandwich system is a method that achieves high specificity and sensitivity by sandwiching a detection target substance between two types of antibodies that recognize different epitopes.

When constructing a sandwich system, it is preferable that at least one of the two types of monoclonal antibodies is a solid-phase antibody, and at least one is preferably a labeled antibody. In the context of the present specification, the "solid-phase antibody" means a monoclonal antibody directly or indirectly immobilized on a solid phase. In the context of the present specification, the "labeled antibody" means a monoclonal antibody that is directly or indirectly labeled with a conventional labeling substance well known in the art described below when measuring a signal attributable to the labeling substance.

For example, a solid-phase antibody can be produced by allowing a monoclonal antibody to physically adsorb or chemically bind to a solid phase (optionally via an appropriate spacer). The solid phase to be used may be a solid phase composed of a polymer substrate such as polystyrene resin, an inorganic substrate such as glass, a polysaccharide substrate such as cellulose or agarose, or the like. The shape of the solid phase is not particularly limited, and any appropriate shape may be chosen, for example, from a plate shape (e.g., microplate or membrane), a bead or particulate shape (e.g., latex particles, magnetic particles), or a cylindrical shape (e.g., test tube).

The use of a labeled antibody (secondary antibody) capable of directly binding to the monoclonal antibody used in the immunoassay assay method of the present invention also enables the measurement of amount of the antibody bound to SARS-CoV-2 or a peptide fragment thereof. In the present specification, an antibody which is bound to the monoclonal antibody of the present invention and has a labeling substance bound thereto is referred to as a "secondary antibody". This secondary antibody may be used to indirectly bind the labeling substance to the monoclonal antibody of the present invention.

By measuring the intensity of the signal generated by the labeling substance, the amount of SARS-CoV-2 or a peptide fragment thereof in the biological sample can be measured. Examples of labeling substances for preparing the labeled antibody include metal complexes, enzymes, insoluble particles, fluorescent substances, chemiluminescent substances, biotin, avidin, radioactive isotopes, colloidal gold particles, and colored latex. The method used for binding the labeling substance to the monoclonal antibody may be physical adsorption, glutaraldehyde method, maleimide method, pyridyl disulfide method, or periodic acid method, which are available to those skilled in the art. When an enzyme such as horseradish peroxidase (HRP) or alkaline phosphatase (ALP) is used as a labeling substance, enzymatic activity can be measured using a specific substrate for the enzyme. For example, as the substrate for measuring the enzymatic activity, o-phenylenediamine (OPD) or 3,3',5,5'-tetramethylbenzidine (TMB) can be used when the enzyme is HRP, and p-nitrophenyl phosphate can be used when the enzyme is ALP. When biotin is used as a labeling substance, a monoclonal antibody may be labeled with biotin and reacted with avidin or streptavidin labeled with an enzyme, dye, or fluorescent label (preferably HRP). In the immunoassay method of the present invention, it is preferable to use colloidal gold particles as the labeling substance.

In the context of the present specification, the process of physically or chemically supporting an antigen or antibody on a solid phase or the state of an antigen or antibody being supported on a solid phase is sometimes referred to as "immobilization" or " solid-phase immobilization". The term "assay", "detection" or "measurement" also encompasses proving the presence of SARS-CoV-2 or a peptide fragment thereof and quantification of SARS-CoV-2 or a peptide fragment thereof.

The immunoassay method of the present invention may be, for example, competitive ELISA, which is a competitive method, including steps (1) to (4) as described below. When competitive ELISA is used, the immunoassay method can be performed using a single type of the monoclonal antibody of the present invention.

(1) A step of immobilizing SARS-CoV-2 or a peptide fragment thereof on a solid phase such as a microplate.
(2) A step of adding a biological sample to be assayed to the microplate.
(3) A step of adding an enzyme-labeled monoclonal antibody of the present invention to the microplate.
(4) A step of adding a substrate for the enzyme and measuring a signal derived from the enzymatic reaction.

When competition occurs between SARS-CoV-2 or a peptide fragment thereof in the biological sample and SARS-CoV-2 or a peptide fragment thereof immobilized on the solid phase, the intensity of the signal decreases. Further, the antibody may be labeled with biotin. Streptavidin labeled with HRP or the like may be bound to this biotin. Then, a chromogenic signal generated by addition of OPD as a substrate can be measured.

In the immunoassay method of the present invention, it is preferable to use two types of monoclonal antibodies that recognize different epitopes. For convenience, one of the monoclonal antibodies may be referred to as "first monoclonal antibody", while the other one of the monoclonal antibodies may be referred to as "second monoclonal antibody".

When the first monoclonal antibody is a labeled antibody and the second monoclonal antibody is a solid-phase antibody, the immunoassay method of the present invention may include the following steps (1) to (3).

(1) A step of contacting a biological sample with a first monoclonal antibody having a labeling substance bound thereto to form a first complex (containing SARS-CoV-2 or a peptide fragment thereof, the labeling substance, and the first monoclonal antibody).
(2) A step of contacting the first complex with a second monoclonal antibody to form a second complex (containing SARS-CoV-2 or a peptide fragment thereof, the labeling substance, the first monoclonal antibody, and the second monoclonal antibody).
(3) A step of measuring a signal attributable to the labeling substance.

The second complex contains a labeling substance. For signal measurement, a measurement method well known in the art can be employed depending on the labeling substance. The signal measurement may be performed using a measuring instrument, or may be performed visually.

When using one or two types of monoclonal antibodies, the immunoassay method of the present invention may include, if necessary, a step of pretreating the biological sample and/or a step of comparing the intensity of the obtained signal with a first threshold. Examples of pretreatments include filtration of the biological sample and dilution of the biological sample with a sample diluent. The first threshold may be appropriately set in consideration of sensitivity and type of the biological sample, etc.

The first threshold may be a numerical range. The first threshold being a numerical range means that the specified range include a specific threshold, and the determination of whether the measured value is larger or smaller than the specific threshold allows determination of the presence or absence of a disease.

The first threshold can be, for example, 1.1 × 10²TCID₅₀/mL.

When the first threshold is a numerical range, the first threshold may be in a range between 1.1 × 10²TCID₅₀/mL and 2.0 × 10²TCID₅₀/mL, a range between 1.1 × 10²TCID₅₀/mL and 1.8 × 10²TCID₅₀/mL, or a range between 1.1 × 10²TCID₅₀/mL to 1.5 × 10²TCID₅₀/mL.

The immunoassay method of the present invention may include a step of determining that the subject is infected with SARS-CoV-2 when the signal intensity is lower (higher) than the first threshold, or a step of determining that the subject is not infected with SARS-CoV-2 when the signal intensity is higher (lower) than the first threshold.

The immunoassay method of the present invention can determine the therapeutic efficacy of a specific drug in a subject infected with SARS-CoV-2 based on the measured signal values. In this instance, the immunoassay method of the present invention may further include the following step in addition to the steps described above.

A step of administering a specific drug to a subject and/or comparing the intensity of the signal with a second threshold.

In this instance, the second threshold can be appropriately set in consideration of the sensitivity and the type of biological sample, but may be the measured value of SARS-CoV-2 or a peptide fragment thereof in the subject prior to administration of a specific drug.

The immunoassay method of the present invention may include a step of determining that a specific drug has a therapeutic efficacy when the signal intensity is lower (higher) than the second threshold, or a step of determining that a specific drug has no therapeutic efficacy when the signal intensity is higher (lower) than the second threshold.

In the determination of the therapeutic efficacy, the therapeutic efficacy may be monitored by conducting measurement every few days.

### (Immunoassay method)

The "immunoassay method" is a method of measuring the level of a substance contained in a biological sample using the reaction between an antigen and an antibody. The term "level" encompasses the amount, concentration, or determination of presence or absence of a substance.

Examples of the immunoassay method of the present invention include, but not limited to, electrochemiluminescence immunoassay (ECL method), enzyme-linked immunosorbent assay (ELISA), latex immunoturbidimetric assay (LTIA method), chemiluminescence immunoassay, immunochromatography, and immunofluorescence. The immunoassay method of the present invention is preferably ELISA or immunochromatography, more preferably immunochromatography.

The immunoassay method of the present invention can be an in vivo or in vitro immunoassay method. Further, a sensitizer may also be used to enhance sensitivity.

The immunoassay method can assay SARS-CoV-2 or a peptide fragment thereof contained in a biological sample in an amount equivalent to 1.1 × 10²TCID₅₀/mL or more.

In the immunoassay method of the present invention, the order of adding the monoclonal antibody of the present invention and the biological sample to the measurement system is not limited as long as the effects of the present invention can be achieved. That is, the monoclonal antibody of the present invention may be added to the measurement system before the addition of the biological sample, or simultaneously with the addition of the biological sample, or after the addition of the biological sample.

The measurement procedure and principle are described below for each immunoassay method to be employed. The following descriptions are presented for illustrative purpose only with respect to the measurement procedure and principle for one embodiment of the present invention, and by no means limit the scope of the present invention.

The immunoassay method of the present invention preferably uses the first embodiment of the monoclonal antibody of the present invention as a labeled antibody, and the second embodiment of the monoclonal antibody of the present invention as a solid-phase antibody.

In each of the immunoassay methods described below, methods known in the art including those described above can be used without any limitation with respect to specific methods such as the method for immobilizing the monoclonal antibody on the solid phase, the method for binding the monoclonal antibody to the labeling substance, and the type of labeling substance.

### (Immunochromatography)

Immunochromatography is an immunoassay method that utilizes the behavior that a labeled antibody bound to a target substance to be detected or a labeled antibody flows on a membrane. A general principle in measuring a target substance to be detected by the immunochromatography is as follows.

An antibody against an antigen (target substance to be detected) is immobilized on an insoluble membrane carrier, which is a chromatographic medium, to prepare a detector unit, which is a stationary phase. Then, a conjugate (labeling substance sensitized by an antibody capable of binding to the target substance to be detected) is used as a mobile phase. The target substance to be detected and the conjugate as a mobile phase are allowed to react specifically, and the resulting the target substance bound to the conjugate is allowed to react, in the detector unit as a stationary phase, specifically with the antibody immobilized on the detector unit.

The measurement procedure and principle when the immunochromatography is employed as the immunoassay method of the present invention are as follows.

(1) A biological sample is brought into contact with a sample supply unit for supplying the biological sample.
(2) SARS-CoV-2 or a peptide fragment thereof in the biological sample is allowed to contact the conjugate, thereby forming a first complex (including SARS-CoV-2 or a peptide fragment thereof, the labeling substance, and the first monoclonal antibody). The conjugate is a labeling substance having the first monoclonal antibody bound thereto.
(3) The first complex and the second monoclonal antibody immobilized on the detector unit come into contact, thereby forming a second complex (including SARS-CoV-2 or a peptide fragment thereof, the labeling substance, the first monoclonal antibody, and the second monoclonal antibody).
(4) The intensity of the signal attributable to the labeling substance contained in the conjugate is measured to confirm the formation of the second complex.

Examples of labeling substances include colloidal gold particles, colloidal platinum particles, color latex particles, magnetic particles, and the like. Among these, colloidal gold particles are preferable. Those skilled in the art can appropriately select and adjust the type and particle size of these labeling substances according to the desired sensitivity.

### (ELISA)

Among various immunoassay methods, ELISA using an enzyme label is also preferable because the target can be measured easily and quickly. In the context of the present specification, the "ELISA" means a method in which an antigen or antibody as a target substance to be detected contained in a sample is trapped with an antibody or antigen corresponding to the target substance, and then detected using an enzymatic reaction. The solid phase is preferably a plate (immunoplate). HRP or ALP can be used as a label.

The measurement procedure and principle when the sandwich ELISA is used as the immunoassay method of the present invention are as follows.

(1) When a biological sample is added to a solid phase on which a solid-phase antibody is immobilized and allowed to react, SARS-CoV-2 or a peptide fragment thereof in the biological sample binds to the solid-phase antibody to form a complex of the solid-phase antibody and the SARS-CoV-2 or the peptide fragment thereof on the solid phase.
(2) When a labeled antibody that recognizes another labeled epitope is added to the solid phase and allowed to react, the labeled antibody binds to the trapped SARS-CoV-2 or peptide fragment thereof and forms a sandwich with the above complex of the solid-phase antibody and the SARS-CoV-2 or the peptide fragment thereof.
(3) After washing, the resulting is reacted with an enzyme substrate to develop color, and the absorbance is measured.

The amount of SARS-CoV-2 or a peptide fragment thereof in the biological sample can be measured according to the amount of labeling substance measured.

A secondary antibody can also be used in the sandwich ELISA. The use of a secondary antibody can amplify the reaction and enhance the detection sensitivity. In the case of an example given below, the secondary antibody is an antibody that specifically recognizes the primary antibody (second monoclonal antibody).

When using a secondary antibody, the following procedures (1) to (5) can be adopted.

(1) A biological sample that has been appropriately treated and diluted is added to a solid phase with a first monoclonal antibody immobilized thereon, followed by incubation, removal of the biological sample and washing.
(2) The primary antibody (second monoclonal antibody) is added, followed by incubation and washing.
(3) Further, an enzyme-labeled secondary antibody is added and incubated.
(4) A substrate is added for color development.
(5) The amount of SARS-CoV-2 or peptide fragments thereof is assayed by measuring color development using a plate reader or the like.

### (Electrochemiluminescence immunoassay)

Electrochemiluminescence immunoassay means a method in which a labeling substance is caused to emit light by application of electric current, and the amount of light emitted is detected to measure the amount of a target substance to be detected. A ruthenium complex can be used as a labeling substance in the electrochemiluminescence immunoassay. An electrode is placed on a solid phase (such as a microplate), and radicals are generated on the electrode to excite the ruthenium complex to emit light. Then, the amount of light emitted from this ruthenium complex can be detected.

The measurement procedure and principle when using the magnetic particles as the solid phase, and the ruthenium complex as the labeling substance are as follows.

(1) When the magnetic particles on which the solid-phase antibody is immobilized are brought into contact with the biological sample, SARS-CoV-2 or a peptide fragment thereof in the biological sample binds to the solid-phase antibody.
(2) When the labeled antibody is brought into contact with the magnetic particles after washing, the labeled antibody binds to the SARS-CoV-2 or the peptide fragment thereof bound to the magnetic particles.
(3) After the magnetic particles are washed, the application of electric current causes light emission depending on the amount of the labeled antibody bound to the SARS-CoV-2 or the peptide fragment thereof. By measuring the amount of luminescence, the amount of the target substance to be detected in the biological sample can be accurately measured.

### (Latex immunoturbidimetry)

Latex immunoturbidimetry is an immunoassay method that utilizes agglutination of an antibody bound to the surface of latex and a target substance to be detected (antigen). The latex particles are not particularly limited as long as they are latex particles generally used for in vitro diagnostic agents. With respect to the latex particles during the agglutination reaction measurement, the concentration, average particle size and the like can be appropriately set according to the sensitivity or performance.

The measurement procedure and principle when the latex immunoturbidimetry is used as the immunoassay method of the present invention are as follows.

(1) A first monoclonal antibody and a second monoclonal antibody are bound to latex particles and brought into contact with a biological sample.
(2) SARS-CoV-2 or a peptide fragment thereof in the biological sample binds to the first monoclonal antibody and the second monoclonal antibody, resulting in agglutination of the antibody-bound latex particles.
(3) The biological sample is irradiated with near-infrared light to measure absorbance or scattered light. Based on the measurements, the antigen concentration can be determined.

When the latex immunoturbidimetry is used as the immunoassay method of the present invention, the latex is a solid phase and acts as a labeling substance as well. That is, both the first monoclonal antibody and the second monoclonal antibody are bound to each of the solid phase and the labeling substance.

### 2. Kit for assaying SARS-CoV-2 in biological sample

The kit of the present invention for assaying SARS-CoV-2 in a biological sample (hereinafter, also referred to simply as "immunoassay kit of the present invention") includes the monoclonal antibody of the present invention.

The immunoassay kit of the present invention may be an immunoassay kit including one type of the monoclonal antibody of the present invention for use in a competitive method, preferably competitive ELISA.

In the immunoassay kit of the present invention, SARS-CoV-2 is preferably assayed using two types of monoclonal antibodies that bind to peptide fragments of the nucleocapsid protein of SARS-CoV-2. In this instance, the two monoclonal antibodies preferably recognize different epitopes. The two types of monoclonal antibodies may be placed in separate containers.

Examples of the immunoassay kit of the present invention include, but not limited to, immunoassay kits for performing immunochromatography, ELISA, electrochemiluminescence immunoassay, latex immunoturbidimetry, chemiluminescence immunoassay, and immunofluorescence. The immunoassay kit of the present invention is preferably an immunoassay kit for performing ELISA or immunochromatography, and more preferably an immunoassay kit for performing immunochromatography.

The immunoassay kit of the present invention can be an immunoassay kit for assaying in vivo or in vitro samples.

The immunoassay kit of the present invention can also include other test reagents such as standard antigen substances and quality control antigen samples, specimen diluents, and/or an instruction manual, etc. A person skilled in the art can appropriately adjust the concentrations of the antibody-containing reagent and the like.

The reagents contained in the kit are described below for each immunoassay method to be employed. The immunoassay kit of the present invention preferably uses the first embodiment of the monoclonal antibody of the present invention as a labeled antibody, and the second embodiment of the monoclonal antibody of the present invention as a solid-phase antibody.

When using immunochromatography, the immunoassay kit of the present invention may take a form in which an immunochromatography test strip is stored and mounted in a suitable container (housing).

The immunochromatography test strip may be composed of a sample pad having a sample supply unit, an insoluble membrane carrier as a chromatography medium, and an absorbent pad disposed at the downstream end of the insoluble membrane carrier.

A detection unit with a first monoclonal antibody immobilized thereon may be placed on the insoluble membrane carrier, and a conjugate pad with a conjugate placed thereon may be placed between the sample pad and the insoluble membrane carrier.

The conjugate may be placed on the sample pad or the insoluble membrane carrier.

As regards other configurations of immunochromatography, for example, those described in International Publication No. 2018/012517 or International Publication No. 2016/031892 may be appropriately adopted.

Examples of labeling substances include colloidal gold particles, colloidal platinum particles, color latex particles, magnetic particles, and the like. Among these, colloidal gold particles are preferable. Those skilled in the art can appropriately adjust the types and particle sizes of these labeling substances.

When using sandwich ELISA, the immunoassay kit of the present invention may include the following (A) and (B): (A) a labeling reagent including a conjugate of a second monoclonal antibody and an enzyme (HRP, ALP, etc.); and (B) a solid phase having a first monoclonal antibody immobilized thereon.

In such a kit, first, a biological sample is added to a solid phase with a first monoclonal antibody immobilized thereon, followed by incubation, removal of the biological sample, and washing. Next, a labeling reagent is added, followed by incubation and addition of a substrate for color development. Thereafter, SARS-CoV-2 or peptide fragments thereof can be assayed by measuring color development using a plate reader or the like.

For competitive ELISA, the immunoassay kit of the present invention may include the following (A) and (B): (A) a solid phase having SARS-CoV-2 or its peptide fragments immobilized thereon, and (B) the monoclonal antibody of the present invention labeled with an enzyme. The solid phase and SARS-CoV-2 or its peptide fragments may be separately included in the immunoassay kit. In this instance, the immobilization of SARS-CoV-2 or peptide fragments including peptide fragments thereof on a solid phase is carried out by an assay performer.

Biotin can also be used. Biotin may be bound to streptavidin leveled with HRP, etc. OPD may further be included as a substrate.

When using the electrochemiluminescence immunoassay, the immunoassay kit of the present invention may include the following (A) and (B).

(A) A labeling reagent including a conjugate of a second monoclonal antibody and an electrochemiluminescent substance (e.g., a ruthenium complex, etc.).
(B) A solid phase having immobilized thereon a first monoclonal antibody that binds to SARS-CoV-2 or a peptide fragment thereof.

For example, in a kit using magnetic particles as the solid phase, the biological sample is added to the magnetic particles on which the first monoclonal antibody that binds to SARS-CoV-2 or a peptide fragment thereof is immobilized and allowed to react, followed by removing the biological sample and washing. The conjugate is then added and allowed to react. After washing the magnetic particles, electrical energy is applied to cause light emission. Thereafter, SARS-CoV-2 or a peptide fragment thereof can be assayed by measuring the amount of light emitted from the labeling substance.

When using the latex immunoturbidimetry, the immunoassay kit of the present invention may include the following (1) and (2).

(1) Latex particles having a first monoclonal antibody bound thereto.
(2) Latex particles having a second monoclonal antibody bound thereto.

When the immunoassay kit of the present invention is a kit for latex immunoturbidimetry, the latex serves as a solid phase and a labeling substance as well. Thus, both the first monoclonal antibody and the second monoclonal antibody are bound to each of the solid phase and the labeling substance.

Thus, the descriptions are given above separately for various aspects of the invention, but the descriptions, definitions of terms, and embodiments described for a particular aspect are also applicable to the other aspects.

Hereinbelow, the present invention will be described in detail with reference to examples, which however should not be construed as limiting the present invention. In the following description, the unit "%" refers to "% by mass", unless otherwise specified.

### [Examples]

### [Preparation Example 1 Preparation of monoclonal antibody]

SARS-CoV-2 (COVID-19) nucleocapsid protein, His-tag 1-419 aa (NUN-C5227 manufactured by ACROBiosystems) (hereinafter referred to as "Nu antigen") was used as an immunogen. The Nu antigen was mixed at 1: 1 with Freund's Complete Adjuvant (Difco Laboratories) for the first immunization and with Freund's Complete Adjuvant (Difco Laboratories) for the second and subsequent immunizations. For Balb/c, subcutaneous immunization was continuously carried out every other week with an immunogen amount of 20 µg for the initial round of immunization, and 10 µg (diluted with PBS) for the second and subsequent rounds of immunization. The blood antibody titers were evaluated by antigen-immobilized ELISA after implementing the third round of immunization. Individuals showing a sufficient increase in titer were intraperitoneally immunized with an immunogen diluted with PBS one to three days before dissection. Then, spleen cells, iliac lymph node cells and inguinal lymph node cells were collected and fused with myeloma cell SP2/0 by electrofusion method. The fused cells were cultured on a 96-well plate, and the culture supernatant was collected 7 or 8 days after the fusion. Thereafter, screening was performed by antigen-immobilized ELISA described below, and strains showing reactivity to the Nu antigen but not to NHis-cBSA were selected. In this process, the medium was exchanged on the day before the screening.

### [Preparation Example 2 Screening of anti-Nu antigen antibody (antigen-immobilized ELISA)]

The Nu antigen and NHis-cBSA (His-tag antigen conjugated to BSA (in-house preparation), 100 ng/mL in PBS) were dispensed into a 96-well plate for ELISA (NUNC442404) (50 µL/well) and allowed to stand at room temperature for 2 hours. After washing 3 times with PBST (400 µL/well), a blocking solution (1% BSA-PBST) was dispensed (100 µL/well) and the resulting was allowed to stand at room temperature for 1 hour or at 4 °C overnight. After removing the blocking solution, a cell culture supernatant (2-fold diluted) and an antiserum (1000- and 10000-fold diluted) were dispensed (50 µL/well) and the resulting was allowed to stand at room temperature for 1 hour. After washing 3 times with PBST, Goat anti-Mouse IgG (H+L) PAb-HRP (1031-05 manufactured by Southern Biotech, 9500-fold diluted) was dispensed (50 µL/well) and the resulting was allowed to stand at room temperature for 1 hour. After washing 3 times with PBST, an OPD coloring solution was dispensed (50 µL/well) and the resulting was allowed to stand at room temperature for 10 minutes. A stop solution was dispensed (50 µL/well), and after stopping the reaction, measurement was performed using a plate reader (Abs. 492 nm). Antibodies that showed reactivity to the Nu antigen but showed no reactivity to the NHis-cBSA were selected. Based on the reactivity, etc., the selected antibodies were classified into Group A (3 types), Group B (15 types), Group C1 (8 types), Group C2 (3 types), Group C3 (3 types), Group C4 (1 type), and Group D (1 type).

### [Assay Example 1 Antibody classification by sandwich ELISA]

The following procedure was followed to investigate whether the Nu antigen could be detected by performing sandwich ELISA using two types of antibodies selected from the obtained antibodies.

One type of the antibody was dispensed into a 96-well plate for ELISA (NUNC442404) and allowed to stand at room temperature for 2 hours. After washing 3 times with PBST, a blocking solution (1% BSA-PBST) was dispensed (100 µL/well) and the resulting was allowed to stand at 4 °C overnight. After removing the blocking solution, the Nu antigen was dispensed and the resulting was allowed to stand at room temperature for 30 minutes. After washing 3 times with PBST, another type of the antibody labeled with biotin was added and the resulting was allowed to stand at room temperature for 1 hour. After washing 3 times with PBST, 50 µL/well of streptavidin-HRP diluted 5,000 times was dispensed and the resulting was allowed to stand at room temperature for 30 minutes. After washing 3 times with PBST, an OPD coloring solution was dispensed and the resulting was allowed to stand at room temperature for 10 minutes. A reaction stop solution was dispensed, and the absorbance was measured using a plate reader (wavelength: 492 nm).

When the Nu antigen could not be detected by sandwich ELISA, the antigen recognition sites of the two types of antibodies were judged to be close, and the two types of antibodies were classified into the same group. Table 1 below shows whether or not antibody pairs in each group could be sandwiched.

**[Table 1]**

| | | Solid-phase antibody | | | | | | |
|---|---|---|---|---|---|---|---|---|
| | | Group A | Group B | Group C1 | Group C2 | Group C3 | Group C4 | Group D |
| Liquid-phase antibody | Group A | - | + | + | + | + | + | + |
| | Group B | + | - | + | + | + | + | + |
| | Group C1 | + | + | - | - | + | - | + |
| | Group C2 | - | + | - | - | + | + | + |
| | Group C3 | + | + | + | + | - | - | + |
| | Group C4 | - | + | - | + | - | - | + |
| | Group D | + | - | - | - | - | + | - |

| | | | | | | | | |
|---|---|---|---|---|---|---|---|---|
| + : Sandwichable, - : Not sandwichable | | | | | | | | |

### [Assay Example 2 Antibody classification by immunochromatography]

An immunochromatography test strip was prepared by the following procedure.
1) Preparation of colloidal gold-labeled antibody solution
   1 mL of phosphate buffer containing 25 µg/mL of anti-SARS-CoV-2 antibody was added to 20 mL of 1 OD/mL-colloidal gold solution, and the resulting mixture was stirred at room temperature for 10 minutes. Subsequently, 2 mL of 10 % BSA solution was added to the colloidal gold solution and the resulting was stirred at room temperature for 5 minutes. The obtained solution was centrifuged at 10,000 rpm for 45 minutes at 10 °C, and the supernatant was removed. The residue was suspended in Conjugate Dilution Buffer (Scripps) to obtain a gold colloid-labeled antibody solution.
2) Preparation of conjugate pad
   The colloidal gold-labeled antibody solution prepared in 1) was diluted to 4 OD/mL with a 1.33 % casein, 4 % sucrose solution (pH 7.5) to prepare a conjugate solution. The conjugate solution was applied in lines onto a glass fiber pad and dried to obtain a conjugate pad.
3) Preparation of antibody-immobilized membrane
   PBS containing 0.75 mg/mL anti-SARS-CoV-2 antibody and 2.5 % sucrose was prepared and used as a test line coating solution. PBS containing 1.0 mg/mL goat anti-mouse IgG monoclonal antibody and 2.5 % sucrose was prepared and used as a control line coating solution. Using a dispenser for immunochromatography "XYZ3050" (manufactured by BioDot), the test line coating solution and the control line coating solution were each applied on a nitrocellulose membrane at 1.0 µL/cm and dried to obtain an antibody-immobilized membrane.
4) Fabrication of test device
   The antibody-immobilized membrane, the conjugate pad, and an absorbent pad were attached to a plastic adhesive sheet, and the resultant was cut into a 5 mm-width strip to obtain an immunochromatography test strip.

### Test Method

### 1) Sample

SARS-CoV-2 (Isolate: USA-WA1/2020) Culture Fluid (Heat Inactivated) (ZeptoMetrix) was dilited to 5.0 × 10⁵TCID₅₀/mL with Universal Transport Medium (BD). The resulting was further 11-fold diluted with a sample diluent for rapid tester FLU/NEXT (Sekisui Medical Co., Ltd.).

### 2) Test procedure

120 µL of the sample was dropped onto the test strip, and 10 minutes later, it was visually determined whether the test line on the antibody-immobilized membrane had developed color.

The results of detection tests for inactivated SARS-CoV-2 antigens using the test strips prepared with various antibody combinations are as shown in Table 2. It was found that the immunochromatography was able to detect the inactivated SARS-CoV-2 antigen in some antibody group pairs that could be sandwiched by ELISA.

**[Table 2]**

| | | Solid-phase antibody | | | | | | |
|---|---|---|---|---|---|---|---|---|
| | | Group A | Group B | Group C1 | Group C2 | Group C3 | Group C4 | Group D |
| Labeled antibody | Group A | - | - | - | - | - | - | - |
| | Group B | - | - | - | - | - | - | - |
| | Group C1 | - | - | - | - | + | - | - |
| | Group C2 | - | + | - | - | ++ | ++ | - |
| | Group C3 | - | - | - | + | - | - | - |
| | Group C4 | - | - | - | + | - | - | - |
| | Group D | - | - | - | - | - | + | - |

| | | | | | | | | |
|---|---|---|---|---|---|---|---|---|
| ++ : Sandwichable plus high sensitibity, + : Sandwichable, - : Not sandwichable | | | | | | | | |

### [Example 1 Antibody-epitope analysis 1]

From each of the antidoby groups, one type of antibody was selected, and it was investigated whether the selected antibodies react with the N-terminal side or the C-terminal side of the Nu antigen.

An anti-His-tag antibody adjusted to 5 µg/mL was dispensed at 50 µL/well into a 96-well ELISA microplate and allowed to stand at 4 °C overnight. After washing 3 times with PBST, 100 µL/well of PBST (blocking solution) containing 1% BSA was dispensed and the resulting was allowed to stand at 4 °C overnight. After removing the blocking solution, the Nu antigen prepared to 100 ng/ml, SARS-CoV-2 (COVID-19) NP NTD domain V2 Recombinant Protein His-tag, 44-180 aa (hereinafter referred to as "NTD-side domain antigen", ProSci 92 -749), or Recombinant nucleoprotein (C-term) antigen for COVID-19 (NP-CTD), His-tag 212-417 aa (hereinafter referred to as "CTD-side domain antigen", rekom biotech, RAG0071) was dispensed at 50 µL/ml, and the resulting was allowed to stand at room temperature for 1 hour. After washing 3 times with PBST, each biotin-labeled antibody adjusted to 1 µg/ml was dispensed at 50 µL/well and the resulting was allowed to stand at room temperature for 1 hour. After washing 3 times with PBST, 50 µL/well of streptavidin-HRP diluted 5,000 times was dispensed and the resulting was allowed to stand at room temperature for 30 minutes. After washing 3 times with PBST, an OPD coloring solution was dispensed and the resulting was allowed to stand at room temperature for 10 minutes. A reaction stop solution was dispensed, and the absorbance was measured using a plate reader (wavelength: 492 nm).

The results are shown in Table 3 below.

**[Table 3]**

| Group | Antibody | Nu antigen | NTD-side domain antigen | CTD-side domain antigen |
|---|---|---|---|---|
| A | S32201 | 0.680 | 4.177 | 0.012 |
| B | S32202 | 0.585 | 0.019 | 4.236 |
| C1 | S32212 | 1.949 | 0.006 | 4.281 |
| C2 | S32213 | 0.679 | 0.006 | 4.209 |
| C3 | S32217 | 1.935 | 0.003 | 4.379 |
| C4 | S32209 | 0.609 | 0.003 | 4.298 |
| D | S32205 | 0.357 | 4.177 | 0.014 |

The antibodies belonging to Groups A and D reacted with the NTD-side region antigen and recognized the N-terminal side of the Nu antigen. The antibodies belonging to Groups B and C reacted with the CTD-side region antigen and recognized the C-terminal side of the Nu antigen. In both Tables 1 and 2, the combinations for which sandwiching was possible were the combination of antibodies belonging Groups B and C, and the combinations of antibodies belonging the same Group C, which suggested that using two types of antibodies that recognize the C-terminal side of the Nu antigen enabled high-sensitivity detection of the Nu antigen.

### [Example 2 Antibody-epitope analysis 2]

Antigen-immoblized ELISA was performed for more detailed epitope analysis with respect to antibodies that react with the C-terminal side of the Nu antigen.

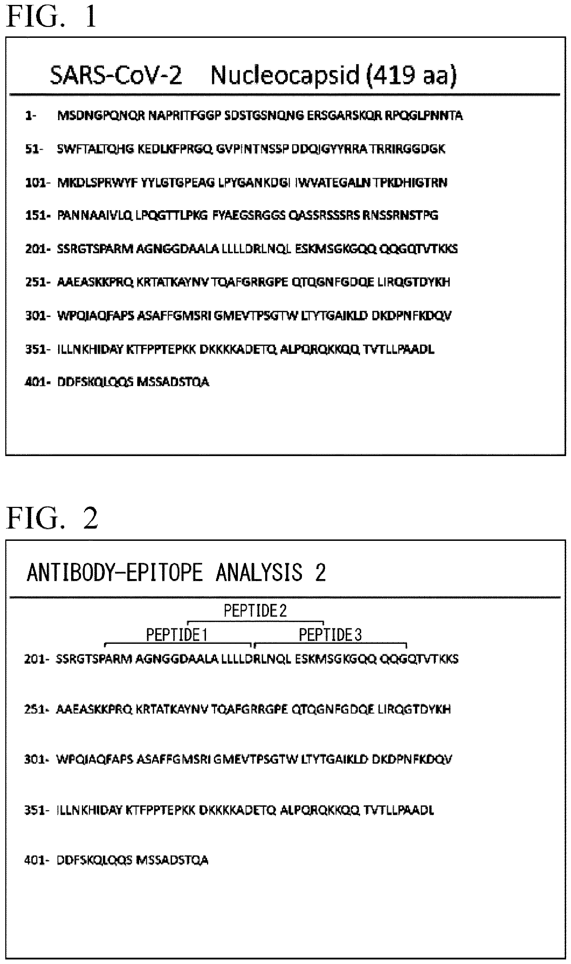

Each of synthetic peptides (10 µg/mL) having amino acid sequences corresponding to specific amino acid sequences of the Nu antigen (see FIG. 2) was dispensed into a 96-well ELISA microplate (50 µL/well) and allowed to stand at room temperature for 2 hours or 4 °C overnight. After washing 3 times with PBST, a blocking solution (1% BSA-PBST) was dispensed (100 µL/well) and the resulting was allowed to stand at room temperature for 1 hour or at 4 °C overnight. After removing the blocking solution, biotin-labeled S32202, S32213, S32212, S32217, or S32209 antibody (1 µg/mL) was dispensed (50 µL/well) and the resulting was allowed to stand at room temperature for 1 hour. After washing 3 times with PBST (400 µL/well), streptavidin-HRP (×5000) was dispensed (50 µL/well) and the resulting was allowed to stand at room temperature for 1 hour. After washing 3 times with PBST (400 µL/well), an OPD coloring solution (2 mg/mL) was dispensed (50 µL/well) and the resulting was allowed to stand at room temperature for 10 minutes. The reaction stop solution was dispensed (50 µL/well), and the absorbance was measured using a plate reader (wavelength: 492 nm).

Table 4 shows the reactivity of antibodies representing the respective groups with synthetic peptides. Those that reacted are marked "+", and those that did not react are marked "-". All antibodies reacted with a sequence of the 388th to 414th amino acids in the nucleocapsid protein (SEQ ID NO: 21). The antibodies belonging to Groups C1 and C2 reacted with a sequence of the 388th to 405th amino acids in the nucleocapsid protein (SEQ ID NO: 51). The antibodies belonging to Groups B, C3, and C4 reacted with a sequence of the 397th to 414th amino acids (SEQ ID NO: 52).

**[Table 4]**

| | | | B | C1 | C2 | C3 | C4 |
|---|---|---|---|---|---|---|---|
| Position | Amino acid sequence | SEQ ID NO: | S32202 | S32212 | S32213 | S32217 | S32209 |
| 208-225 | ARMAGNGGDAALALLLLD | SEQ ID NO: 31 | - | - | - | - | - |
| 217-234 | AALALLLLDRLNQLESKM | SEQ ID NO: 32 | - | - | - | - | - |
| 226-243 | RLNQLESKMSGKGQQQQG | SEQ ID NO: 33 | - | - | - | - | - |
| 235-252 | SGKGQQQQGQTVTKKSAA | SEQ ID NO: 34 | - | - | - | - | - |
| 244-261 | QTVTKKSAAEASKKPRQK | SEQ ID NO: 35 | - | - | - | - | - |
| 253-270 | EASKKPRQKRTATKAYNV | SEQ ID NO: 36 | - | - | - | - | - |
| 262-279 | RTATKAYNVTQAFGRRGP | SEQ ID NO: 37 | - | - | - | - | - |
| 271-288 | TQAFGRRGPEQTQGNFGD | SEQ ID NO: 38 | - | - | - | - | - |
| 280-297 | EQTQGNFGDQELIRQGTD | SEQ ID NO: 39 | - | - | - | - | - |
| 289-306 | QELIRQGTDYKHWPQIAQ | SEQ ID NO: 40 | - | - | - | - | - |
| 298-315 | YKHWPQIAQF APSASAFF | SEQ ID NO: 41 | - | - | - | - | - |
| 307-324 | FAPSASAFFGMSRIGM EV | SEQ ID NO: 42 | - | - | - | - | - |
| 316-333 | GMSRIGMEVTPSGTWLTY | SEQ ID NO: 43 | - | - | - | - | - |
| 325-342 | TPSGTWLTYTGAIKLDDK | SEQ ID NO: 44 | - | - | - | - | - |
| 334-351 | TGAIKLDDKDPNFKDQVI | SEQ ID NO: 45 | - | - | - | - | - |
| 343-360 | DPNFKDQVILLNKHIDAY | SEQ ID NO: 46 | - | - | - | - | - |
| 352-369 | LLNKHIDAYKTFPPTEPK | SEQ ID NO: 47 | - | - | - | - | - |
| 361-378 | KTFPPTEPKKDKKKKADE | SEQ ID NO: 48 | - | - | - | - | - |
| 370-387 | KDKKKKADETQALPQRQK | SEQ ID NO: 49 | - | - | - | - | - |
| 379-396 | TQALPQRQKKQQTVTLLP | SEQ ID NO: 50 | - | - | - | - | - |
| 388-405 | KQQTVTLLPAADLDDFSK | SEQ ID NO: 51 | - | + | + | - | - |
| 397-414 | AADLDDFSKQLQQSMSSA | SEQ ID NO: 52 | + | - | - | + | + |
| 406-419 | QLQQSMSSADSTQA | SEQ ID NO: 53 | - | - | - | - | - |

### [Example 3 Antibody-epitope analysis 3]

Detailed epitope analyses for the S32213 and S32217 antibodies were performed using PEPperMAP (registered trademark) Peptide Microarray analysis service provided by PEPperPRINT. Tables 5 and 6 show excerpts of the analysis results of linear epitope mapping (peptide chain length of 15 amino acid residues, analyzed using 14 amino acid residue overlapping peptides), and conformational epitope mapping (peptide chain length of 7, 10, and 13 amino acid residues, analyzed using 6, 9, and 12 amino acid residue overlapping peptides, respectively). The S32213 antibody was shown to recognize LLPAA (SEQ ID NO: 25), which is a sequence of the 394th to 398th amino acids of the SARS-CoV-2 nucleocapsid protein, and the S32217 antibody was found to recognize LDDFSKQLQ (SEQ ID NO: 29), which is a sequence of the 400th to 408th amino acids of the same protein.

**[Table 5]**

| Position | Amino acid sequence | SEQ ID NO: | Reactivity with S32213 antibody |
|---|---|---|---|
| 385-391 | RQKKQQT | SEQ ID NO: 54 | - |
| 386-392 | QKKQQTV | SEQ ID NO: 55 | - |
| 387-393 | KKQQTVT | SEQ ID NO: 56 | - |
| 388-394 | KQQTVTL | SEQ ID NO: 57 | - |
| 389-395 | QQTVTLL | SEQ ID NO: 58 | - |
| 390-396 | QTVTLLP | SEQ ID NO: 59 | - |
| 391-397 | TVTLLPA | SEQ ID NO: 60 | + |
| 392-398 | VT**LLPAA** | SEQ ID NO: 61 | +++ |
| 393-399 | T**LLPAA**D | SEQ ID NO: 62 | +++ |
| 394-400 | **LLPAA**DL | SEQ ID NO: 63 | +++ |
| 395-401 | LPAADLD | SEQ ID NO: 64 | + |
| 396-402 | PAADLDD | SEQ ID NO: 65 | - |
| 397-403 | AADLDDF | SEQ ID NO: 66 | - |
| 398-404 | ADLDDFS | SEQ ID NO: 67 | - |
| 399-405 | DLDDFSK | SEQ ID NO: 68 | - |
| 400-406 | LDDFSKQ | SEQ ID NO: 69 | - |

| | | | |
|---|---|---|---|
| +++ : Reacted with high sensitivity, + : Reacted, - : Not reacted | | | |

**[Table 6]**

| Position | Amino acid sequence | SEQ ID NO: | Reactivity with S32217 antibody |
|---|---|---|---|
| 389-403 | QQTVTLLPAADLDDF | SEQ ID NO: 70 | - |
| 390-404 | QTVTLLPAADLDDFS | SEQ ID NO: 71 | - |
| 391-405 | TVTLLPAADLDDFSK | SEQ ID NO: 72 | - |
| 392-406 | VTLLPAADLDDFSKQ | SEQ ID NO: 73 | - |
| 393-407 | TLLPAADLDDFSKQL | SEQ ID NO: 74 | + |
| 394-408 | LLPAAD**LDDFSKQLQ** | SEQ ID NO: 75 | ++ |
| 395-409 | LPAAD**LDDFSKQLQ**Q | SEQ ID NO: 76 | ++ |
| 396-410 | PAAD**LDDFSKQLQ**QS | SEQ ID NO: 77 | ++ |
| 397-411 | AAD**LDDFSKQLQ**QSM | SEQ ID NO: 78 | ++ |
| 398-412 | AD**LDDFSKQLQ**QSMS | SEQ ID NO: 79 | ++ |
| 399-413 | D**LDDFSKQLQ**QSMSS | SEQ ID NO: 80 | ++ |
| 400-414 | **LDDFSKQLQ**QSMSSA | SEQ ID NO: 81 | +++ |
| 401-415 | DDFSKQLQQSMSSAD | SEQ ID NO: 82 | + |
| 402-416 | DFSKQLQQSMSSADS | SEQ ID NO: 83 | - |
| 403-417 | FSKQLQQSMSSADST | SEQ ID NO: 84 | - |
| 404-418 | SKQLQQSMSSADSTQ | SEQ ID NO: 85 | - |

| | | | |
|---|---|---|---|
| +++ : Reacted with particularly high sensitivity, ++ : Reacted with high sensitivity, + : Reacted, - : Not reacted | | | |

### [Example 4 Antibody reactivity with trypsin-digested Nu antigen]

Trypsin derived from porcine pancreas was diluted with PBS to prepare a trypsin solution (0 or 2000 ng/mL). Each trypsin solution was mixed with the PBS-diluted Nu antigen (2000 ng/mL) at a ratio of 1:1, and reacted at 37 °C for 10 minutes. After the reaction, a protease inhibitor was added in an amount of 1/100 of the solution to stop the enzymatic reaction, and the resulting was used as trypsin-digested Nu antigen.

Five types of test antibodies (5 µg/mL) were dispensed into a 96-well ELISA microplate (50 µL/well) and allowed to stand at room temperature for 2 hours. After washing 3 times with PBST, a blocking solution (1% BSA-PBST) was dispensed (100 µL/well) and the resulting was allowed to stand at room temperature for 1 hour. After removing the blocking solution, the trypsin-digested Nu antigen (1000 ng/ml) was dispensed (50 µL/ml) and the resulting was allowed to stand at room temperature for 30 minutes. After washing 3 times with PBST (400 µL/well), synthetic peptides (2 µg/mL, 25 µL/well) and 5 types of biotin-labeled test antibodies (1 µg/ml) were dispensed (50 µL/well), and the resulting was allowed to stand at room temperature. After washing 3 times with PBST (400 µL/well), streptavidin-HRP (×5000) was dispensed (50 µL/well) and the resulting was allowed to stand at room temperature for 1 hour. After washing 3 times with PBST (400 µL/well), an OPD coloring solution (2 mg/mL) was dispensed (50 µL/well) and the resulting was allowed to stand at room temperature for 10 minutes. The reaction stop solution was dispensed (50 µL/well), and the absorbance was measured using a plate reader (wavelength: 492 nm). The results are shown in Table 7.

**[Table 7]**

| Liquid-phase antibody | S32213 (C2) | S32217 (C3) | S32212 (C1) | S32209 (C4) | S32231 (B) |
|---|---|---|---|---|---|
| Solid-phase antibody | S32217 (C3) | S32213 (C2) | S32217 (C3) | S32213 (C2) | S32217 (C3) |
| Without trypsin digestion | 4.245 | 4.369 | 4.634 | 4.229 | 4.409 |
| With trypsin digestion | 4.595 | 4.284 | 4.456 | 4.511 | 0.122 |

With respect to the combination of antibodies belonging to Group C, the Nu antigen could be detected even when trypsin digestion was performed. However, as for the combination of Group B and Group C3, tryptic digestion significantly reduced the antigen-antibody reaction. The antibodies belonging to Group B presumably recognize a certain amino acid sequence in QLQQSMSSA (SEQ ID NO: 27) as an epitope.

### [Example 5 Detection of SARS-CoV-2 by immunochromatography]

An immunochromatography test strip was prepared in the same manner as in Assay Example 1. S32213 antibody was used as a labeled antibody, and S32217 antibody was used as a solid-phase antibody.

### 5) Test method

500 µL each of a sample diluent for rapid tester FLU/NEXT (Sekisui Medical Co. Ltd.) was dispensed into diluent tubes. Universal Transport Medium (BD) was added to SARS-CoV-2 PCR positive swab (Trina) to prepare a sample. A sample collection cotton swab was inserted into the sample to collect the sample, and then the sample was extracted with the diluent in the diluent tube. A sample filtration filter was attached to the diluent tube, and the entire amount was filtered. The above sample was added dropwise in an amount of 120 µL per drop onto the test device, and 10 minutes later, the presence or absence of the test line was visually determined.

RNA was extracted from the same sample using QIAmp Viral RNA Mini Kit (QIAGEN). RT-PCR was performed following "Pathogen Detection Manual 2019-nCoV Ver. 2.9.1", National Institute of Infectious Diseases. The results are shown in Table 8. In this context, the RT-PCR results showed that 29 samples were positive and 26 samples were negative. Samples that were positive at the time of sample collection were purchased and used for the tests, some of which however became negative, presumably due to the effects of transportation, freezing and thawing, and sample dilution.

**[Table 8]**

| | | Immunochromatography in Example | | Total |
|---|---|---|---|---|
| | | Positive | Negative | |
| RT-PCR | Positive | 29 | 0 | 29 |
| | Negative | 2 | 24 | 26 |
| Total | | 31 | 24 | 55 |

The positive percent agreement with RT-PCR in the immunochromatography was 100 % (29/29 × 100). The negative percent agreement with RT-PCR in the immunochromatography was 92.3 % (24/26 × 100). The number of copies of SARS-CoV-2 per test was investigated for the samples used. All 29 positive samples contained 1600 or more copies of SARS-CoV-2 per test.

In this context, the package insert of the commercially available SARS-CoV-2 antigen detection reagents describe the positive percent agreement when measuring a sample with 1600 copies/test.

Espline (registered trademark) SARS-CoV-2 (Fujirebio Inc.) had a positive percent agreement of 92 % (12/13 × 100) with a measurement time of 30 minutes.

QuickNavi (registered trademark)-COVID19 Ag (Denka Company Limited) had a positive percent agreement of 96.3 % (26/27 × 100) with a measurement time of 15 minutes.

Therefore, the immunochromatography of this example showed sensitivity comparable to or greater than that of the commercially available reagent for SARS-CoV-2 antigen detection with a shorter measurement time. Therefore, it can be said that SARS-CoV-2 can be detected rapidly and with high sensitivity by using the immunochromatography of this example.

### [Example 6 Specificity evaluation for immunochromatography]

The same test device as used in Example 5 was used. 500 µL each of the sample diluent for Rapid Tester FLU·NEXT was dispensed into diluent tubes, and the sample was extracted from two nasopharyngeal swab sticks used for the same healthy individual per diluent tube. A sample filtration filter was attached to the diluent tube, and the entire amount was filtered. The above sample was added dropwise in an amount of 120 µL per drop onto the same test device as used in Example 5, and 10 and 30 minutes later, visual and machine determinations were made. Further, RT-PCR was also performed in the same manner as in Example 5. Table 9 shows the determination results for 30 samples which were respectively derived from different donors.

**[Table 9]**

| Sample | Determination | | |
|---|---|---|---|
| | Immunochromatography in Example | | RT-PCR |
| | Measurement time: 10 min | Measurement time: 30 min | |
| 1 | - | - | - |
| 2 | - | - | - |
| 3 | - | - | - |
| 4 | - | - | - |
| 5 | - | - | - |
| 6 | - | - | - |
| 7 | - | - | - |
| 8 | - | - | - |
| 9 | - | - | - |
| 10 | - | - | - |
| 11 | - | - | - |
| 12 | - | - | - |
| 13 | - | - | - |
| 14 | - | - | - |
| 15 | - | - | - |
| 16 | - | - | - |
| 17 | - | - | - |
| 18 | - | - | - |
| 19 | - | - | - |
| 20 | - | - | - |
| 21 | - | - | - |
| 22 | - | - | - |
| 23 | - | - | - |
| 24 | - | - | - |
| 25 | - | - | - |
| 26 | - | - | - |
| 27 | - | - | - |
| 28 | - | - | - |
| 29 | - | - | - |
| 30 | - | - | - |

All samples 1 to 30 were found to be negative by RT-PCR. In 10 minutes after starting the immunochromatography in the example, all the samples were again found to be negative. Further, all the samples were also found to be negative in 30 minutes after starting the immunochromatography in the example, which exceeded the predetermined measurement time for the immunochromatography in the example. Thus, the immunochromatography in this example showed good specificity.

### [Example 7 Analysis of amino acid sequence of monoclonal antibody]

The amino acid sequences of the heavy chain variable region and light chain variable region of the S32213 antibody, S32217 antibody, and S32223 antibody were analyzed using the antibody variable region analysis of Kazusa DNA Research Institute, a public interest incorporated foundation. As a result, the amino acid sequences of the heavy chain variable region and the light chain variable region were respectively found to be as shown in Table 10 below.

**[Table 10]**

| Name of antibody | Heavy chain or light chain | CDR | Amino acid sequence | SEQ ID NO: |
|---|---|---|---|---|
| S32213 | Heavy chain | CDR1 | GFSLSTSGMG | SEQ ID NO: 3 |
| | | CDR2 | IYWDDDK | SEQ ID NO: 4 |
| | | CDR3 | ARRDYGYNFDY | SEQ ID NO: 5 |
| | Light chain | CDR1 | SSVSSTY | SEQ ID NO: 6 |
| | | CDR2 | STS | SEQ ID NO: 7 |
| | | CDR3 | HQWSSYPPT | SEQ ID NO: 8 |
| S32217 | Heavy chain | CDR1 | GFTFSDYY | SEQ ID NO: 9 |
| | | CDR2 | ITDGDNYT | SEQ ID NO: 10 |
| | | CDR3 | ARDGNYYASSPFTY | SEQ ID NO: 11 |
| | Light chain | CDR1 | TGAVTTSNY | SEQ ID NO: 12 |
| | | CDR2 | GTN | SEQ ID NO: 13 |
| | | CDR3 | ALWYSNHWV | SEQ ID NO: 14 |
| S32223 | Heavy chain | CDR1 | GFTFSDYY | SEQ ID NO: 15 |
| | | CDR2 | ISDGYSYT | SEQ ID NO: 16 |
| | | CDR3 | ARDQDYFGSSLAY | SEQ ID NO: 17 |
| | Light chain | CDR1 | TGAVTTSNY | SEQ ID NO: 18 |
| | | CDR2 | GTN | SEQ ID NO: 19 |
| | | CDR3 | ALWYSNRWV | SEQ ID NO: 20 |

As in the case of the S32217 antibody, the S32223 antibody also belongs to the Group C3 in the antibody classification by sandwich ELISA in Assay Example 1. The amino acid sequences of CDR1 to CDR3 in the heavy chain variable region and light chain variable region of the S32223 antibody respectively have high identity with the amino acid sequences of CDR1 to CDR3 in the heavy chain variable region and light chain variable region of the S32217 antibody. Therefore, the S32223 antibody is presumed to have similar reactivity to the SARS-CoV-2 antigen as the S32217 antibody.

### [Example 8 Influence of serine protease degradation]

### <Experiment 1> Relationship between trypsin treatment time and measurement sensitivity

An inactivated antigen (Vero cell SARS-CoV-2 infected cell lysate MOI = 3; National Institute of Infectious Diseases) was added to a specimen transportation medium at a concentration of 256 ng/mL, thereby preparing a sample. Trypsin dissolved in PBS was added to the sample to give a final concentration of 1 µg/mL, and the resulting was heated at 37 °C. After reacting for 2, 5, 15, 30 minutes, Protease Inhibitor Cocktail Set I, Animal-derived-free (for general use) (×100) (FUJIFILM Wako Pure Chemical Corporation) was added according to the package insert to stop the reaction, followed by adding 50 µL each of the resulting reaction solution to sample diluent for rapid tester FLU/NEXT (Sekisui Medical Co., Ltd.), Espline (registered trademark) SARS-CoV-2 sample treatment solution (Fujirebio), and Quick Navi (registered trademark) sample suspension (for COVID19 Ag) (Denka Company Limited) to obtain measurement samples.

The measurement samples were measured with the immunochromatography test strip used in Example 5, Espline (registered trademark) SARS-CoV-2 (Fujirebio) and QuickNavi (registered trademark)-COVID19 Ag (Denka Company Limited), each of which is an existing SARS-CoV-2 measurement reagent.

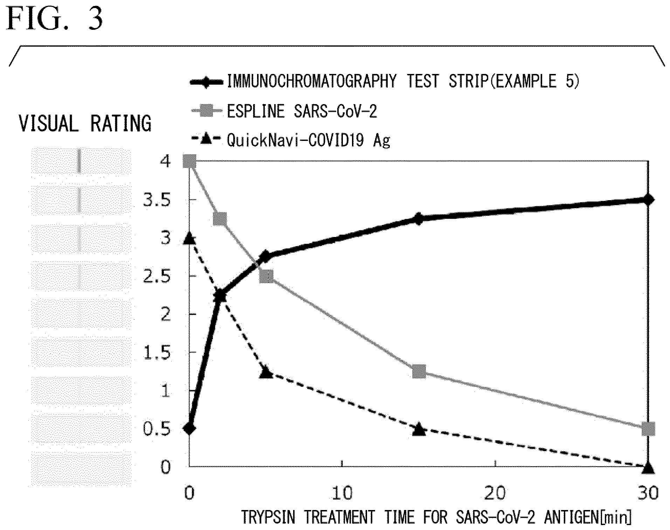

The results are shown in FIG. 3.

As shown in FIG. 3, the existing measurement reagents decreased in measurement sensitivity as the trypsin treatment time increased, and their measurement sensitivity for the sample treated with trypsin for 30 minutes was 20 % or less relative to that for the sample before the trypsin treatment, whereas the measurement reagent of the present invention (Example 5) increased in measurement sensitivity as the trypsin treatment time increased.

### <Experiment 2> Relationship between nasal swab treatment time and measurement sensitivity

Nasal swabs collected from three healthy individuals with cotton swabs were suspended in 500 µL of PBS per three cotton swabs and pooled. A recombinant antigen (SARS-CoV-2 (COVID-19) Nucleocapsid protein, His Tag; manufactured by ACROBiosystems) was added to the nasal swab suspension pool at a concentration of 1 µg/mL to prepare a sample. After heating the sample at 37 °C to perform reaction for 30 and 60 minutes, Protease Inhibitor Cocktail set I, Animal-derived-free (for general purpose) (x 100) (FUJIFILM Wako Pure Chemical Corporation) was added according to the package insert to stop the reaction, followed by adding 10 µL each of the resulting reaction solution to sample diluent for Rapid Tester FLUINEXT (Sekisui Medical Co., Ltd.) and QuickNavi (registered trademark) sample suspension (for COVID19 Ag) (Denka Company Limited) to obtain measurement samples.

The measurement samples were measured with the immunochromatography test strip used in Example 5 and QuickNavi (registered trademark)-COVID19 Ag (Denka Company Limited) which is an existing SARS-CoV-2 measurement reagent.

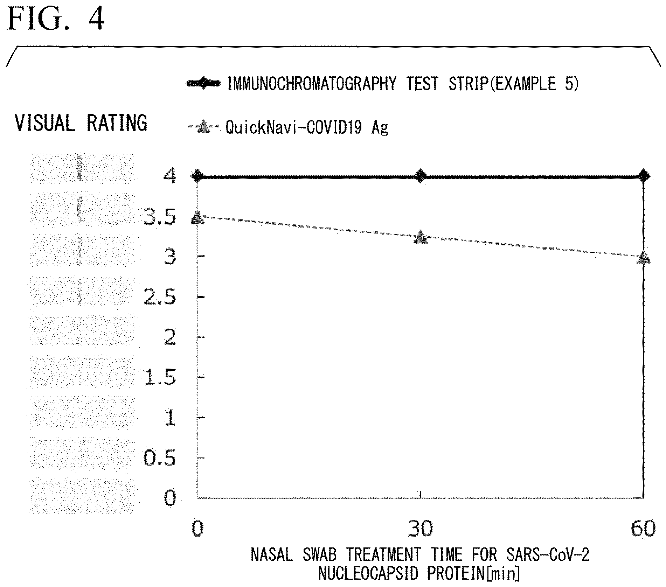

The results are shown in FIG. 4.

As shown in FIG. 4, the measurement sensitivity of the existing SARS-CoV-2 measurement reagent decreased depending on the nasal swab treatment time, whereas the measurement sensitivity of the measurement reagent of the present invention (Example 5) did not change.

### <Explanation of results>

The results of Experiment 1 show that the immunoassay method of the present invention can detect SARS-CoV-2 degraded by a serine protease.

Further, the results of Experiment 2 show that the immunoassay method of the present invention can detect nucleocapsid proteins that have been degraded by serine proteases expressed in organs such as tracheal epithelium.

Thus, the results of Experiments 1 and 2 have shown that, by using two monoclonal antibodies that bind to peptide fragments generated from SARS-CoV-2 degraded with a serine protease such as trypsin, the immunoassay method of the present invention enables implementation of high-sensitivity immunoassay for SARS-CoV-2.

### [List of Sequences]

**[Table 11]**

| SEQ ID NO: | Amino acid sequence (N terminus → C terminus) |
|---|---|
| 1 | KQQTVTLLPAADLDDFSK |
| 2 | AADLDDFSKQLQ |
| 3 | GFSLSTSGMG |
| 4 | IYWDDDK |
| 5 | ARRDYGYNFDY |
| 6 | SSVSSTY |
| 7 | STS |
| 8 | HQWSSYPPT |
| 9 | GFTFSDYY |
| 10 | ITDGDNYT |
| 11 | ARDGNYYASSPFTY |
| 12 | TGAVTTSNY |
| 13 | GTN |
| 14 | ALWYSNHWV |
| 15 | GFTFSDYY |
| 16 | ISDGYSYT |
| 17 | ARDQDYFGSSLAY |
| 18 | TGAVTTSNY |
| 19 | GTN |
| 20 | ALWYSNRWV |

**[Table 12]**

| SEQ ID NO: | Amino acid sequence (N terminus → C terminus) |
|---|---|
| 21 | MSDNGPQNQR NAPRITFGGP SDSTGSNQNG ERSGARSKQR RPQGLPNNTA SWFTALTQHG KEDLKFPRGQ GVPINTNSSP DDQIGYYRRA TRRIRGGDGK MKDLSPRWYF YYLGTGPEAG LPYGANKDGI IWVATEGALN TPKDHIGTRN PANNAAIVLQ LPQGTTLPKG FYAEGSRGGS QASSRSSSRS RNSSRNSTPG SSRGTSPARM AGNGGDAALA LLLLDRLNQL ESKMSGKGQQ QQGQTVTKKS AAEASKKPRQ KRTATKAYNV TQAFGRRGPE QTQGNFGDQE LIRQGTDYKH WPQIAQFAPS ASAFFGMSRI GMEVTPSGTW LTYTGAIKLD DKDPNFKDQV ILLNKHIDAY KTFPPTEPKK DKKKKADETQ ALPQRQKKQQ TVTLLPAADL DDFSKQLQQS MSSADSTQA |
| 22 | MSDNGPQNQR NAPRITFGGP SDSTGSNQNG ERSGARSKQR RPQGLPNNTA SWFTALTQHG KEDLKFPRGQ GVPINTNSSP DDQIGYYRRA TRRIRGGDGK MKDLSPRWYF YYLGTGPEAG LPYGANKDGI IWVATEGALN TPKDHIGTRN PANNAAIVLQ LPQGTTLPKG FYAE |
| 23 | GSRGGSQASS RSSSRSRNSS RNSTPGSSRG TSPARMAGNG GDAALALLLL DRLNQLESKM SGKGQQQGQ TVT |
| 24 | KKSAAEASKK PRQKRTATKA YNVTQAFGRR GPEQTQGNFG DQELIRQGTD YKHWPQIAQF APSASAFFGM SRIGMEVTPS GTWLTYTGAI KLDDKDPNFK DQVILLNKHI DAYKTFPPTE PKKDKKKKAD ETQALPQRQK KQQTVTLLPA ADLDDFSKQL QQSMSSADST QA |

**[Table 13]**

| SEQ ID NO: | Amino acid sequence (N terminus → C terminus) |
|---|---|
| 25 | LLPAA |
| 26 | LDDFSKQLQ |
| 27 | QLQQSMSSA |
| 28 | KQQTVTLLPAADLDDFSKQLQ |
| 29 | LDDFSKQLQ |
| 30 | AADLDDFSKQLQQSMSSA |
| 31 | ARMAGNGGDAALALLLLD |
| 32 | TAALALLLLDRLNQLESKM |
| 33 | RLNQLESKMSGKGQQQQG |
| 34 | SGKGQQQQGQTVTKKSAA |
| 35 | QTVTKKSAAEASKKPRQK |
| 36 | EASKKPRQKRTATKAYNV |
| 37 | RTATKAYNVTQAFGRRGP |
| 38 | TQAFGRRGPEQTQGNFGD |
| 39 | EQTQGNFGDQELIRQGTD |
| 40 | QELIRQGTDYKHWPQIAQ |
| 41 | YKHWPQIAQFAPSASAFF |
| 42 | FAPSASAFFGMSRIGMEV |
| 43 | GMSRIGMEVTPSGTWLTY |
| 44 | TPSGTWLTYTGAIKLDDK |
| 45 | TGAIKLDDKDPNFKDQVI |
| 46 | DPNFKDQVILLNKHIDAY |
| 47 | LLNKHIDAYKITFPPTEPK |
| 48 | KTFPPTEPKKDKKKKADE |
| 49 | KDKKKKADETQALPQRQK |
| 50 | TQALPQRQKKQQTVTLLP |
| 51 | KQQTVTLLPAADLDDFSK |
| 52 | AADLDDFSKQLQQSMSSA |
| 53 | QLQQSMSSADSTQA |

**[Table 14]**

| SEQ ID NO: | Amino acid sequence (N terminus → C terminus) |
|---|---|
| 54 | RQKKQQT |
| 55 | QKKQQTV |
| 56 | KKQQTVT |
| 57 | KQQTVTL |
| 58 | QQTVTLL |
| 59 | QTVTLLP |
| 60 | TVTLLPA |
| 61 | VTLLPAA |
| 62 | TLLPAAD |
| 63 | LLPAADL |
| 64 | LPAADLD |
| 65 | PAADLDD |
| 66 | AADIDDF |
| 67 | ADLDDFS |
| 68 | DLDDESK |
| 69 | LDDFSKQ |

**[Table 15]**

| SEQ ID NO: | Amino acid sequence (N terminus → C terminus) |
|---|---|
| 70 | QQTVTLLPAADLDDF |
| 71 | QTVTLLPAADLDDFS |
| 72 | TVTLLPAADLDDFSK |
| 73 | VTLLPAADLDDESKQ |
| 74 | TLLPAADLDDFSKQL |
| 75 | LLPAADLDDESKQLQ |
| 76 | LPAADLDDFSKQLQQ |
| 77 | PAADLDDFSKQLQQS |
| 78 | AADLDDFSKQLQQSM |
| 79 | ADLDDFSKQLQQSMS |
| 80 | DLDDFSKQLQQSMSS |
| 81 | LDDFSKQLQQSMSSA |
| 82 | DDFSKQLQQSMSSAD |
| 83 | DFSKQLQQSMSSADS |
| 84 | FSKQLQQSMSSADST |
| 85 | SKQLQQSMSSADSTQ |

### [Industrial Applicability]

The present invention can provide a SARS-CoV-2 immunoassay kit and a SARS-CoV-2 immunoassay method that enable highly sensitive and rapid immunoassay, as well as a monoclonal antibody or an antibody fragment thereof that can be used therein.

## Claims

1. An immunoassay method for assaying SARS-CoV-2, comprising contacting a biological sample with a monoclonal antibody or an antibody fragment thereof that binds to a nucleocapsid protein of SARS-CoV-2,
wherein the monoclonal antibody or the antibody fragment thereof binds to SARS-CoV-2 treated with a serine protease.

2. The immunoassay method according to claim 1, wherein the monoclonal antibody or the antibody fragment thereof recognizes an epitope in an amino acid sequence represented by KQQTVTLPAADLDDFSK (SEQ ID NO: 1) or an epitope in an amino acid sequence represented by AADLDDFSKQLQ (SEQ ID NO: 2).

3. The immunoassay method according to claim 1, wherein the biological sample is treated with a serine protease.

4. The immunoassay method according to claim 1 or 2, wherein the biological sample is a respiratory secretion.

5. The immunoassay method according to claim 1 or 2, wherein the monoclonal antibody or the antibody fragment comprises two types of monoclonal antibodies or antibody fragments thereof that bind to peptide fragments of a nucleocapsid protein of SARS-CoV-2, wherein:
the two types of monoclonal antibodies or antibody fragments thereof are a first monoclonal antibody or an antibody fragment thereof and a second monoclonal antibody or an antibody fragment thereof, wherein the first monoclonal antibody or the antibody fragment thereof has a labeling substance indirectly or directly bound thereto, and the second monoclonal antibody or the antibody fragment thereof is indirectly or directly bound to a solid phase; and
the first monoclonal antibody or the antibody fragment thereof and the second monoclonal antibody or the antibody fragment thereof recognize different epitopes.

6. The immunoassay method according to claim 5, wherein, as step (1), the biological sample is contacted with the first monoclonal antibody or the antibody fragment thereof having the labeling substance bound thereto, thereby forming a first complex, and which further comprises step (2) of contacting the first complex with the second monoclonal antibody or the antibody fragment thereof to form a second complex, and step (3) of measuring a signal derived from the labeling substance.

7. The immunoassay method according to claim 5, wherein the first monoclonal antibody or the antibody fragment thereof is a monoclonal antibody or an antibody fragment thereof that recognizes an epitope in the amino acid sequence represented by KQQTVTLLPAADLDFSK (SEQ ID NO: 1), and the second monoclonal antibody or the antibody fragment thereof is a monoclonal antibody or an antibody fragment thereof that recognizes an epitope in the amino acid sequence represented by AADLDDFSKQLQ (SEQ ID NO: 2).

8. The immunoassay method according to claim 1 or 2, which is immunochromatography or ELISA.

9. The immunoassay method according to claim 1 or 2, wherein the monoclonal antibody or the antibody fragment thereof is at least one selected from the group consisting of:
(1) an antibody or an antibody fragment thereof that comprises a heavy chain variable region comprising CDR1 having an amino acid sequence of SEQ ID NO: 3, CDR2 having an amino acid sequence of SEQ ID NO: 4, and CDR3 having an amino acid sequence of SEQ ID NO: 5, and a light chain variable region comprising CDR1 having an amino acid sequence of SEQ ID NO: 6, CDR2 having an amino acid sequence of SEQ ID NO: 7, and CDR3 having an amino acid sequence of SEQ ID NO: 8;
(2) an antibody or an antibody fragment thereof that comprises a heavy chain variable region comprising CDR1 having an amino acid sequence of SEQ ID NO: 9, CDR2 having an amino acid sequence of SEQ ID NO: 10, and CDR3 having an amino acid sequence of SEQ ID NO: 11, and a light chain variable region comprising CDR1 having an amino acid sequence of SEQ ID NO: 12, CDR2 having an amino acid sequence of SEQ ID NO: 13, and CDR3 having an amino acid sequence of SEQ ID NO: 14;
(3) an antibody or an antibody fragment thereof that comprises a heavy chain variable region comprising CDR1 having an amino acid sequence of SEQ ID NO: 15, CDR2 having an amino acid sequence of SEQ ID NO: 16, and CDR3 having an amino acid sequence of SEQ ID NO: 17, and a light chain variable region comprising CDR1 having an amino acid sequence of SEQ ID NO: 18, CDR2 having an amino acid sequence of SEQ ID NO: 19, and CDR3 having an amino acid sequence of SEQ ID NO: 20; and
(4) an antibody or an antibody fragment thereof that comprises a heavy chain variable region and a light chain variable region respectively having 80 % or more amino acid sequence identity with the heavy chain variable region and the light chain variable region of the antibody or the antibody fragment thereof in any one of (1) to (3).

10. An immunoassay kit for assaying SARS-CoV-2 in a biological sample, comprising a monoclonal antibody or an antibody fragment thereof that binds to a nucleocapsid protein of SARS-CoV-2, wherein the monoclonal antibody or the antibody fragment thereof binds to the SARS-CoV-2 treated with a serine protease.

11. The immunoassay kit according to claim 10, wherein the monoclonal antibody or the antibody fragment thereof recognizes an epitope in an amino acid sequence represented by KQQTVTLPAADLDDFSK (SEQ ID NO: 1) or an epitope in an amino acid sequence represented by AADLDDFSKQLQ (SEQ ID NO: 2).

12. The immunoassay kit according to claim 10 or 11, wherein the biological sample is treated with a serine protease.

13. The immunoassay kit according to claim 10 or 11, wherein the biological sample is a respiratory secretion.

14. The immunoassay kit according to claim 10 or 11, wherein the monoclonal antibody or the antibody fragment comprises two types of monoclonal antibodies or antibody fragments thereof that bind to peptide fragments of a nucleocapsid protein of SARS-CoV-2, wherein:
the two types of monoclonal antibodies or antibody fragments thereof are a first monoclonal antibody or an antibody fragment thereof and a second monoclonal antibody or an antibody fragment thereof, wherein the first monoclonal antibody or the antibody fragment thereof has a labeling substance indirectly or directly bound thereto, and the second monoclonal antibody or the antibody fragment thereof is indirectly or directly bound to a solid phase; and
the first monoclonal antibody or the antibody fragment thereof and the second monoclonal antibody or the antibody fragment thereof recognize different epitopes.

15. The immunoassay kit according to claim 14, wherein the first monoclonal antibody or the antibody fragment thereof is a monoclonal antibody or an antibody fragment thereof that recognizes an epitope in the amino acid sequence represented by KQQTVTLLPAADLDFSK (SEQ ID NO: 1), and the second monoclonal antibody or the antibody fragment thereof is a monoclonal antibody or an antibody fragment thereof that recognizes an epitope in the amino acid sequence represented by AADLDDFSKQLQ (SEQ ID NO: 2).

16. The immunoassay kit according to claim 10 or 11, which is a kit for immunochromatography or ELISA.

17. The immunoassay kit according to claim 10 or 11, wherein the monoclonal antibody or the antibody fragment thereof is at least one selected from the group consisting of:
(1) an antibody or an antibody fragment thereof that comprises a heavy chain variable region comprising CDR1 having an amino acid sequence of SEQ ID NO: 3, CDR2 having an amino acid sequence of SEQ ID NO: 4, and CDR3 having an amino acid sequence of SEQ ID NO: 5, and a light chain variable region comprising CDR1 having an amino acid sequence of SEQ ID NO: 6, CDR2 having an amino acid sequence of SEQ ID NO: 7, and CDR3 having an amino acid sequence of SEQ ID NO: 8;
(2) an antibody or an antibody fragment thereof that comprises a heavy chain variable region comprising CDR1 having an amino acid sequence of SEQ ID NO: 9, CDR2 having an amino acid sequence of SEQ ID NO: 10, and CDR3 having an amino acid sequence of SEQ ID NO: 11, and a light chain variable region comprising CDR1 having an amino acid sequence of SEQ ID NO: 12, CDR2 having an amino acid sequence of SEQ ID NO: 13, and CDR3 having an amino acid sequence of SEQ ID NO: 14;
(3) an antibody or an antibody fragment thereof that comprises a heavy chain variable region comprising CDR1 having an amino acid sequence of SEQ ID NO: 15, CDR2 having an amino acid sequence of SEQ ID NO: 16, and CDR3 having an amino acid sequence of SEQ ID NO: 17, and a light chain variable region comprising CDR1 having an amino acid sequence of SEQ ID NO: 18, CDR2 having an amino acid sequence of SEQ ID NO: 19, and CDR3 having an amino acid sequence of SEQ ID NO: 20; and
(4) an antibody or an antibody fragment thereof that comprises a heavy chain variable region and a light chain variable region respectively having 80 % or more amino acid sequence identity with the heavy chain variable region and the light chain variable region of the antibody or the antibody fragment thereof in any one of (1) to (3).

18. A monoclonal antibody or an antibody fragment thereof that binds to a nucleocapsid protein of SARS-CoV-2, wherein the monoclonal antibody or the antibody fragment thereof binds to SARS-CoV-2 treated with a serine protease.

19. The monoclonal antibody or the antibody fragment thereof according to claim 18, which recognizes an epitope in an amino acid sequence represented by KQQTVTLLPAADLDDFSK (SEQ ID NO: 1) or an epitope in an amino acid sequence represented by AADLDDFSKQLQ (SEQ ID NO: 2).

20. The monoclonal antibody or the antibody fragment thereof according to claim 18 or 19, which is selected from the group consisting of
(1) an antibody or an antibody fragment thereof that comprises a heavy chain variable region comprising CDR1 having an amino acid sequence of SEQ ID NO: 3, CDR2 having an amino acid sequence of SEQ ID NO: 4, and CDR3 having an amino acid sequence of SEQ ID NO: 5, and a light chain variable region comprising CDR1 having an amino acid sequence of SEQ ID NO: 6, CDR2 having an amino acid sequence of SEQ ID NO: 7, and CDR3 having an amino acid sequence of SEQ ID NO: 8;
(2) an antibody or an antibody fragment thereof that comprises a heavy chain variable region comprising CDR1 having an amino acid sequence of SEQ ID NO: 9, CDR2 having an amino acid sequence of SEQ ID NO: 10, and CDR3 having an amino acid sequence of SEQ ID NO: 11, and a light chain variable region comprising CDR1 having an amino acid sequence of SEQ ID NO: 12, CDR2 having an amino acid sequence of SEQ ID NO: 13, and CDR3 having an amino acid sequence of SEQ ID NO: 14;
(3) an antibody or an antibody fragment thereof that comprises a heavy chain variable region comprising CDR1 having an amino acid sequence of SEQ ID NO: 15, CDR2 having an amino acid sequence of SEQ ID NO: 16, and CDR3 having an amino acid sequence of SEQ ID NO: 17, and a light chain variable region comprising CDR1 having an amino acid sequence of SEQ ID NO: 18, CDR2 having an amino acid sequence of SEQ ID NO: 19, and CDR3 having an amino acid sequence of SEQ ID NO: 20; and
(4) an antibody or an antibody fragment thereof that comprises a heavy chain variable region and a light chain variable region respectively having 80 % or more amino acid sequence identity with the heavy chain variable region and the light chain variable region of the antibody or the antibody fragment thereof in any one of (1) to (3).
